# EUROPEAN PATENT APPLICATION

(11) **EP 4 850 601 A1**
(43) Date of publication of application: **09.12.2026**
(21) Application number: 25747561.6
(22) Date of filing: 16.01.2025
(51) Int. Cl.: A61B 5/145

(54) **APPLICATION DEVICE AND ANALYTE MONITORING SYSTEM**

(30) Priority: 02.02.2024 CN 202410151895
(71) Applicant: SHENZHEN SISENSING CO., LTD., Shenzhen Guangdong 518110 (CN)
(72) Inventor: LI, Yunfeng, Shenzhen, Guangdong 518110 (CN)
(74) Representative: Vossius & Partner Patentanwälte Rechtsanwälte mbB
(86) International application number: PCT/CN2025/072869
(87) International publication number: WO 2025/161989

(57) **Abstract**

An applicator device and an analyte monitoring system are disclosed. The applicator device includes a first housing, a second housing, a drive mechanism, a movable body, and a penetrating member disposed on the movable body. The applicator device has a proximal end located proximal to a host during operation and a distal end located distal to the host during operation. The first housing and the second housing are movable toward one another to release retention of the movable body. Once released, the movable body and the penetrating member move together toward the proximal end under a force of the drive mechanism to apply a monitoring device to the host, with at least a portion of the monitoring device positioned beneath the skin of the host. The disclosed device and system are easy to operate and accurately apply a monitoring device to a host.

## Description

### BACKGROUND OF INVENTION

The present disclosure generally relates to the field of medical instruments, and more particularly relates to an applicator device and an analyte monitoring system.

### DESCRIPTION OF RELATED ART

Currently, the primary methods by which diabetic patients monitor their blood glucose levels themselves include conventional Blood Glucose Monitoring (BGM) and Continuous Glucose Monitoring Systems (CGMS). Among these, CGMS can monitor a patient's glucose level in real time and is capable of detecting occult hyperglycemia and hypoglycemia that are not easily detected by conventional finger-stick blood glucose testing methods, thereby becoming a new trend in glucose monitoring.

A CGMS generally uses a glucose sensor that can be inserted beneath the skin and react with glucose in interstitial fluid, together with an electronic device attached to the skin and connected to the glucose sensor, to monitor glucose concentration in the interstitial fluid. To insert the glucose sensor beneath the skin and attach the electronic device to the skin, an insertion device is typically required. Specifically, the insertion device may comprise a needle capable of penetrating the skin, through which the glucose sensor is inserted beneath the skin, after which the needle is separated from the glucose sensor and withdrawn from the skin.

However, in the above-mentioned conventional technologies, insertion of the needle into the skin and withdrawal of the needle from the skin generally require the aid of manual drive. In such cases, since the force applied to drive the needle, the location where the needle penetrates the skin, the depth to which the needle penetrates the skin, or the timing of needle withdrawal from the skin and the like may be difficult to accurately control, on the one hand, the user's experience during insertion of the glucose sensor may be adversely affected, and on the other hand, the accuracy of the obtained glucose concentration information may be reduced because the glucose sensor may not be inserted into a desired position.

### SUMMARY OF INVENTION

The present disclosure has been made in view of the circumstances of the above-described prior art, and an object thereof is to provide an applicator device and an analyte monitoring system that are easy to operate and capable of accurately applying a monitoring device to a host.

To this end, the present disclosure provides an applicator device for applying a monitoring device to a host. The applicator device comprises a first housing, a second housing, a drive mechanism, a movable body, a penetrating member disposed in the movable body, and a proximal end positioned proximal to the host during operation and a distal end positioned distal to the host during operation. The first housing comprises a first retaining portion configured to releasably retain the movable body. The second housing comprises a proximal surface configured to contact the skin of the host during operation. The movable body comprises a receiving portion configured to accommodate the monitoring device. The penetrating member is configured to at least partially introduce the monitoring device into a subcutaneous region of the host. The first housing and the second housing are configured to move relative to each other toward one another so as to release the movable body from retention by the first retaining portion. After being released, the movable body and the penetrating member are moved together toward the proximal end under the action of the drive mechanism so as to apply the monitoring device to the host, with at least a portion of the monitoring device positioned beneath the skin of the host.

In the present disclosure, the applicator device comprises the movable body configured to accommodate the monitoring device, the penetrating member configured to at least partially introduce the monitoring device into a subcutaneous region of the host, the first housing configured to releasably retain the movable body, and the second housing configured to cooperate with the first housing to release the locking of the movable body by the first housing (that is, to cause the first retaining portion to release the movable body, which operation may be referred to as "unlocking"). After unlocking, the movable body and the penetrating member can be moved toward the proximal end, which is proximal to the host, under the action of the drive mechanism, thereby applying the monitoring device to the body surface of the host, and the penetrating member is capable of introducing at least a portion of the monitoring device into the subcutaneous region of the host. In this case, by placing the proximal surface of the second housing against the skin of the host, it becomes easier to apply force to cause relative movement between the two housings (the first housing and the second housing) and thereby unlock the movable body, while also facilitating defining of the application site. Furthermore, the drive mechanism can provide a relatively accurate driving force, thereby capable of more accurately controlling the penetrating force and penetrating depth of the penetrating member during application, which is conducive to accurately applying the monitoring device to the host.

Additionally, in the applicator device according to the present disclosure, the movable body optionally comprises a first locking portion configured to releasably interlock with the first retaining portion, and the second housing comprises a first trigger portion configured to separate the first retaining portion from the first locking portion so as to release the movable body. Accordingly, the movable body may be releasably retained to the first retaining portion through cooperation between the first locking portion and the first retaining portion, and the first trigger portion facilitates release of the movable body through relative movement of the two housings.

Additionally, in the applicator device according to the present disclosure, optionally, when the first housing and the second housing move relative to each other along a direction of a central axis of the applicator device, the first trigger portion acts on the first locking portion so as to separate the first locking portion from the first retaining portion. In this case, simply moving the two housings relative to each other along the direction of the central axis enables the first trigger portion to separate the first locking portion from the first retaining portion, thereby facilitating use by a user.

Additionally, in the applicator device according to the present disclosure, optionally, the first retaining portion is a protruding structure formed on an inner wall of the first housing. When the movable body is retained to the first retaining portion, the first locking portion engages the protruding structure. The first trigger portion causes the first locking portion to deflect in a direction away from the first retaining portion so as to separate the first locking portion from the first retaining portion. In this case, compared with providing, within the first housing, a first retaining portion having a certain length and capable of being displaced, providing the first retaining portion as a protruding structure formed on the inner wall of the first housing can reduce the space required for the first retaining portion while reducing manufacturing complexity of the first housing, thereby facilitating miniaturization of the applicator device and ease of use of an operator.

Additionally, in the applicator device according to the present disclosure, optionally, the first housing comprises a first restricting portion configured to prevent the second housing from disengaging from the first housing. The second housing comprises a first restricted portion coupled to the first restricting portion. In this case, the second housing can be restrained by the first housing through cooperation between the first restricted portion and the first restricting portion, and prevention of disengagement of the second housing from the first housing helps improve the reliability of the applicator device.

Additionally, in the applicator device according to the present disclosure, optionally, the first housing comprises a second restricting portion configured to inhibit movement of the second housing toward the proximal end. After the movable body is released by relative movement of the first housing and the second housing, the second housing is restrained by the second restricting portion. In this case, the second restricting portion can limit movement of the second housing toward the proximal end, thereby preventing repeated use of the applicator device by the operator and reducing the risk that a used penetrating member that has not undergone re-sterilization may come into contact with a host again.

Additionally, in the applicator device according to the present disclosure, optionally,the first restricting portion and the second restricting portion are arranged adjacent to each other along the direction of the central axis of the applicator device, and the first restricted portion is configured to cooperate with the second restricting portion to retain the second housing. This arrangement thus helps simplify the structure of the applicator device.

Additionally, in the applicator device according to the present disclosure, optionally,the drive mechanism comprises a first drive portion and a second drive portion. The movable body comprises a second retaining portion configured to releasably retain the penetrating member. After the movable body and the penetrating member have been moved together toward the proximal end to a predetermined position under the action of the first drive portion, the second retaining portion releases the penetrating member, and the penetrating member is moved toward the distal end under the action of the second drive portion. In this case, the application operation and the retraction operation are respectively performed by the first drive portion and the second drive portion, thereby enabling more accurate control of the penetrating force, penetrating depth, and timing of exiting the host of the penetrating member.

Additionally, in the applicator device according to the present disclosure, optionally, the first drive portion is an elastic element, and opposite ends of the first drive portion respectively abut against the first housing and the movable body. When the movable body is retained by the first retaining portion, the first drive portion is in a compressed state. In this case, when the movable body is released, the first drive portion in the compressed state is capable of automatically exerting a force on the movable body, thereby achieving automatic advancement and facilitating operation by a user.

Additionally, in the applicator device according to the present disclosure, optionally, the second drive portion is an elastic element, and opposite ends of the second drive portion respectively abut against the movable body and the penetrating member. When the penetrating member is retained by the second retaining portion, the second drive portion is in a compressed state. In this case, when the penetrating member is released, the second drive portion in the compressed state is capable of automatically exerting a force on the penetrating member, thereby achieving automatic retraction of the penetrating member and facilitating operation by a user.

Additionally, in the applicator device according to the present disclosure, optionally, the applicator device comprises an applicator cap detachably fitted to the first housing. When the applicator cap is fitted to the first housing, the second housing is located within a sealed accommodation space defined by the applicator cap and the first housing. In this case, cooperation between the applicator cap and the first housing forms a sealed space for protecting internal structures, and placement of the second housing within the sealed space helps prevent unintended actuation, thereby improving the reliability of the applicator device.

Additionally, in the applicator device according to the present disclosure, optionally, the monitoring device comprises a sensor configured to be partially placed beneath the skin of the host, an electronic assembly coupled to the sensor, and a sealing assembly configured to seal the sensor. The applicator cap is coupled to the sealing assembly, and when the applicator cap is removed from the first housing, the sealing assembly is removed together with the applicator cap so that the applicator device is placed in a ready-to-apply state. In this case, the sealing assembly that seals the sensor may help maintain the sterile condition of the sensor for an extended period of time, and the coupling between the applicator cap and the sealing assembly allows the sealing assembly to be removed simultaneously with removal of the applicator cap, thereby facilitating use by an operator.

Additionally, in the applicator device according to the present disclosure, optionally, the sealing assembly comprises a first engagement feature, and the applicator cap comprises a second engagement feature. The first engagement feature cooperates with the second engagement feature such that, when the applicator cap is rotated in a first direction, the sealing assembly rotates together with the applicator cap, and when the applicator cap is rotated in a second direction opposite to the first direction, the sealing assembly remains stationary. In this case, the sealing assembly can be removed simultaneously with removal of the applicator cap by rotating the applicator cap in the first direction, thereby facilitating use. Furthermore, during assembly of the applicator cap onto the first housing by rotating the applicator cap in the second direction opposite to the first direction, the sealing state of the sealing assembly is not affected, thereby facilitating factory assembly of the applicator device and the monitoring device.

According to a second aspect of the present disclosure, an analyte monitoring system is provided, comprising the applicator device described in the first aspect of the present disclosure and a monitoring device. Accordingly, an analyte monitoring system that is easy to operate and capable of accurately applying a monitoring device to a host can be provided.

According to the present disclosure, an applicator device and an analyte monitoring system that are easy to operate and capable of accurately applying a monitoring device to a host can be provided.

### BRIEF DESCRIPTION OF THE DRAWINGS

Embodiments of the present disclosure will now be explained in further detail by way of example only with reference to the accompanying drawings.
FIG. 1 is a schematic view illustrating an application scenario of a monitoring device according to an example of the present disclosure.
FIG. 2 is a schematic view illustrating a monitoring device according to an example of the present disclosure.
FIG. 3 is a schematic view illustrating a sensor according to an example of the present disclosure.
FIG. 4 is a schematic view illustrating a sterilization assembly according to an example of the present disclosure.
FIG. 5 is an exploded view of the sterilization assembly according to an example of the present disclosure.
FIG. 6A is a schematic view illustrating the sterilization assembly and the electronic assembly before assembly according to an example of the present disclosure.
FIG. 6B is a schematic view illustrating the sterilization assembly and the electronic assembly after assembly according to an example of the present disclosure.
FIG. 7 is an overall perspective view of an applicator device according to an example of the present disclosure.
FIG. 8 is a cross-sectional view of the applicator device according to an example of the present disclosure.
FIG. 9 is an exploded view of the applicator device according to an example of the present disclosure.
FIG. 10 is a schematic view illustrating a first housing according to an example of the present disclosure.
FIG. 11 is a schematic view illustrating a second housing according to an example of the present disclosure.
FIG. 12 is a schematic view illustrating a movable body according to an example of the present disclosure.
FIG. 13 is a cross-sectional schematic view of the first housing, the second housing, and the movable body after assembly according to an example of the present disclosure.
FIG. 14 is a cross-sectional schematic view illustrating a first locking portion before being triggered by a first trigger portion according to an example of the present disclosure.
FIG. 15 is a cross-sectional schematic view illustrating a first locking portion after being triggered by a first trigger portion according to an example of the present disclosure.
FIG. 16 is a cross-sectional schematic view illustrating a first restricted portion not engaged with a second restricting portion according to an example of the present disclosure.
FIG. 17 is a cross-sectional schematic view illustrating the first restricted portion engaged with the second restricting portion according to an example of the present disclosure.
FIG. 18 is an exploded schematic view of the movable body and the penetrating member from a first perspective according to an example of the present disclosure.
FIG. 19 is an exploded schematic view of the movable body and the penetrating member from a second perspective according to an example of the present disclosure.
FIG. 20 is a cross-sectional schematic view illustrating the penetrating member in a retained state according to an example of the present disclosure.
FIG. 21 is a schematic view illustrating the sealing assembly and the applicator cap according to an example of the present disclosure.
FIG. 22 is a schematic view illustrating a structure of an inclined slot according to an example of the present disclosure.

### DETAILED DESCRIPTION

Preferred embodiments of the present disclosure will now be described in detail with reference to the accompanying drawings. In the following description, identical reference numerals designate identical components, and repetitive descriptions thereof are omitted. In addition, the drawings are merely schematic, and the relative dimensional scales between, and shapes of the components may differ from those in an actual implementation.

It should be noted that the terms "comprise(s)" and "have/has," as well as any variations thereof, as used in the present disclosure, are intended to cover a non-exclusive inclusion. For example, a process, method, system, product, or apparatus comprising a series of steps or units is not necessarily limited to the explicitly listed steps or units, but may further comprise other steps or units not expressly listed or inherent to such process, method, system, product, or apparatus.

The present disclosure relates to an applicator device that may be used to apply a monitoring device to a host, for example, by attaching the monitoring device to a body surface of the host or placing the monitoring device wholly or partially beneath the skin of the host and the like. The applicator device according to the present embodiments facilitates application of the monitoring device to a desired location. In the present disclosure, the monitoring device may be used to monitor physiological parameters of the host. The monitoring device may also comprise other medical instruments intended to be applied to a host.

The applicator device described in the present embodiments may also be referred to as an insertion device, delivery device, propulsion device, transport device, implantation device, attachment device, auxiliary device, or the like. It should be understood that these names merely identify a device used to apply a monitoring device to a host and should not be construed as limiting.

Additionally, the present disclosure further provides an analyte monitoring system, which may comprise a monitoring device and an applicator device. The monitoring device may be used to acquire physiological parameter information of a host. The monitoring device may be applied to the host by means of the applicator device, and physiological parameter information of the host may thereafter be monitored using the monitoring device. In the present disclosure, the analyte monitoring system may also be referred to as an analyte monitoring kit, analyte combination kit, or a monitoring system and the like.

The applicator device and analyte monitoring system according to the present disclosure will now be described with reference to the accompanying drawings.

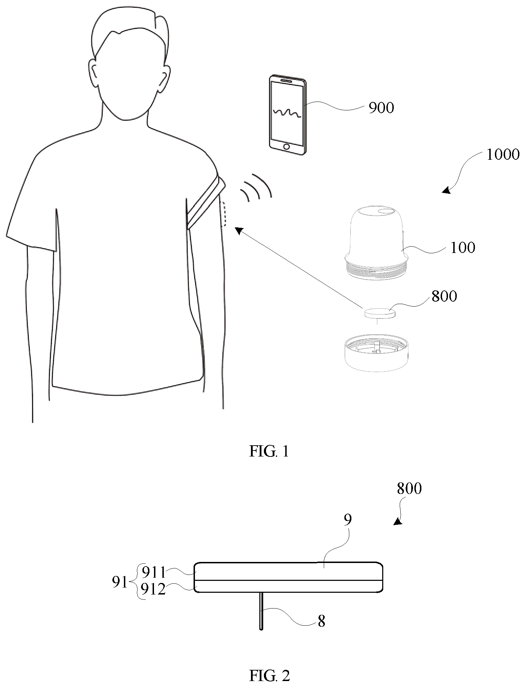

FIG. 1 illustrates an application scenario of a monitoring device 800 according to an example of the present disclosure.

In some examples, the monitoring device 800 may be applied to a host by means of the applicator device 100 and applied to a desired location. The host may acquire physiological parameter information (which may be referred to simply as physiological information) via the monitoring device 800 applied thereto.

In some examples, the analyte monitoring system 1000 may comprise the monitoring device 800 and the applicator device 100 (see FIG. 1). The monitoring device 800 may be configured to acquire physiological parameter information of a host. The monitoring device 800 may be applied to the host by the applicator device 100, and the physiological parameter information of the host may thereafter be monitored via the monitoring device 800.

In some examples, the analyte monitoring system 1000 may further comprise a reader device 900 communicatively connected to the monitoring device 800 (see FIG. 1). The monitoring device 800 applied to the host may transmit acquired physiological parameter information, for example wirelessly, to the reader device 900, thereby facilitating reading and monitoring of the host's physiological parameter information.

In some examples, the monitoring device 800 may be an analyte sensor device configured to generate information regarding a specific analyte in a body fluid based on the body fluid. For example, the monitoring device 800 may react with an analyte present in the body fluid and generate analyte information. In such a case, the sensor 8 may react with the analyte in the body fluid, thereby facilitating acquisition of analyte information from the body fluid.

In some examples, the analyte may be one or more of glucose, acetylcholine, amylase, bilirubin, cholesterol, chorionic gonadotropin, creatine kinase, creatine, creatinine, DNA, fructosamine, glutamine, growth hormone, ketones, lactate, oxygen, peroxide, prostate-specific antigen (PSA), prothrombin, RNA, thyroid-stimulating hormone (TSH), or troponin.

For ease of understanding, the monitoring device 800 of the present disclosure will first be described. The following description uses glucose as an example analyte to explain the monitoring device 800 according to the present embodiments. It should be understood that a person skilled in the art could readily slightly modify the monitoring device 800 configured for glucose monitoring to monitor other analytes.

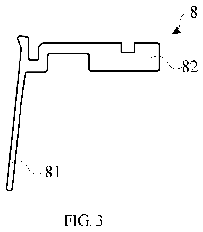

FIG. 2 is a schematic view of the monitoring device 800 according to an example of the present disclosure, and FIG. 3 is a schematic view of the sensor 8 according to an example of the present disclosure.

In some examples, the monitoring device 800 may comprise a sensor 8 and an electronic assembly 9 (see FIG. 2). The sensor 8 may be fitted to the electronic assembly 9. To facilitate interaction with an interstitial fluid, such as body fluid, the sensor 8 may be placed partially or entirely beneath the skin of the host. After being placed subcutaneously, the sensor 8 may react with glucose in the interstitial fluid beneath the skin to generate glucose information of the host. The electronic assembly 9 may be attached to a body surface of the host and electrically connected to the sensor 8 so as to receive the glucose information generated by the sensor 8. In addition, the electronic assembly 9 may provide electrical power required by the sensor 8 to acquire physiological information. Accordingly, continuous subcutaneous monitoring by the sensor 8 may be facilitated.

In some examples, the sensor 8 may comprise an implanted portion 81 and a connection portion 82 (see FIG. 3). The implanted portion 81 may be placed beneath the skin of the host and connected to the electronic assembly 9 through the connection portion 82. In this case, the implanted portion 81 can be placed beneath the skin of the host and connected to the electronic assembly 9 through the connection portion 82, thereby facilitating temporary fixation of the sensor 8 at a particular site on the host.

In some examples, the implanted portion 81 may comprise a working electrode and a counter electrode. In the present embodiment, a sensing layer including glucose oxidase may be provided on the working electrode. After the sensor 8 is placed subcutaneously, glucose oxidase on the working electrode may undergo an oxidation-reduction reaction with glucose in interstitial fluid or blood. A circuit may thereby be formed with the counter electrode to generate an electrical current signal. The current signal may then be analyzed and processed to acquire glucose concentration information. The current signal generated by the sensor 8 may be transmitted to the electronic assembly 9.

In some examples, the implanted portion 81 may further comprise a reference electrode. The reference electrode may establish a known and fixed potential difference relative to interstitial fluid or blood. In such a case, the potential difference between the working electrode and the interstitial fluid or blood may be obtained based on the potential difference formed between the reference electrode and the working electrode, thereby enabling more accurate determination of the voltage generated at the working electrode. In addition, in such a case, the electronic assembly 9 may automatically adjust and maintain the voltage at the working electrode according to a preset voltage value, such that the measured current signal more accurately reflects the glucose concentration in the interstitial fluid or blood. In some examples, one or more reference electrodes may be provided, for example two reference electrodes.

In some examples, the electronic assembly 9 may comprise an enclosure 91 (see FIG. 2). The electronic assembly 9 may further comprise electronic devices provided within the enclosure 91. In some examples, the electronic devices may comprise a power supply module, a switching module, and a processing module. The power supply module may supply electrical power to the sensor 8 and/or the processing module. The switching module may control electrical connection and disconnection between the power supply module and the sensor 8 and/or the processing module. The processing module may process signals generated by the sensor 8 (for example, glucose information).

In some examples, the electronic devices may have an initial mode configured as an open-circuit state and an operating mode configured as a closed-circuit state. In such a case, when the electronic devices are in the initial mode, they are configured in an open-circuit state, thereby conserving power before electronic devices enter an operation state. In some examples, when the electronic devices are configured in the initial mode, the switching module is in an open state, whereas when the electronic devices are configured in the operating mode, the switching module is in a closed state. In other words, the switching module may switch the electronic devices from the initial mode to the operating mode. In this case, controlling the switch from the initial mode to the operating mode via the switching module can effectively reduce power consumption before operation of the electronic devices.

In some examples, the electronic assembly 9 may be configured to be activated from the initial mode to the operating mode by magnetic action or optical action. In this case, the use of non-contact components, such as magnetic or optical action to activate the electronic assembly 9 from the initial mode to the operating mode, facilitates miniaturization of the electronic assembly 9.

In some examples, the applicator device 100 (described later) may comprise a magnet. In some examples, before the monitoring device 800 is applied to a host, the switching module of the electronic assembly 9 may remain in an open state because the electronic assembly 9 is positioned proximate to the magnet. In some examples, when the monitoring device 800 has been applied to the host, the switching module of the electronic assembly 9 may be activated to a closed state because the electronic assembly 9 is then located farther away from the magnet. In such a case, while the analyte monitoring system 1000 is in a standby state after manufacture and before use, the monitoring device 800 is located within the applicator device 100 and may remain in a low-power or zero-power initial mode, thereby helping extend the standby life (also referred to as shelf life) of the monitoring device 800.

In some examples, after the monitoring device 800 has been applied to the host but before the applicator device 100 has been removed from the host, the switching module of the electronic assembly 9 may remain in an open state because the electronic assembly 9 of the monitoring device 800 is still located proximate to the magnet. In some examples, after the monitoring device 800 has been applied to the host and the applicator device 100 has been removed from the host, the switching module of the electronic assembly 9 may be activated to a closed state because the electronic assembly 9 of the monitoring device 800 is then located farther away from the magnet.

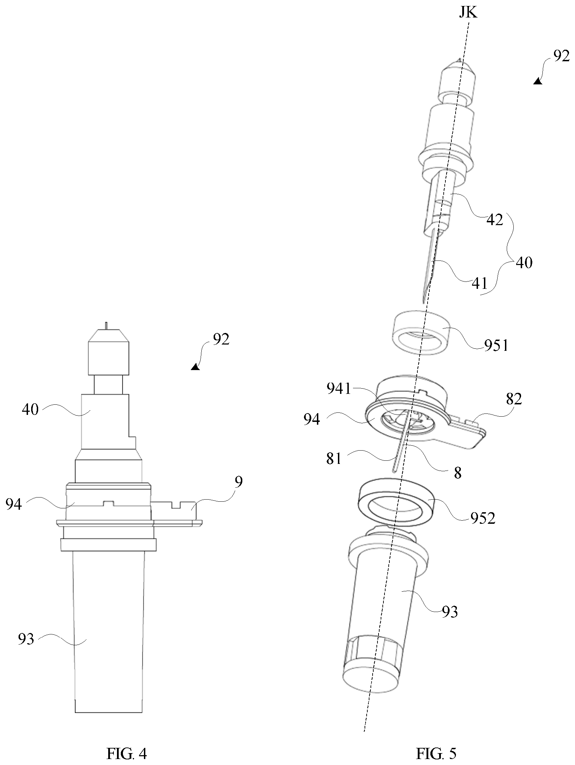

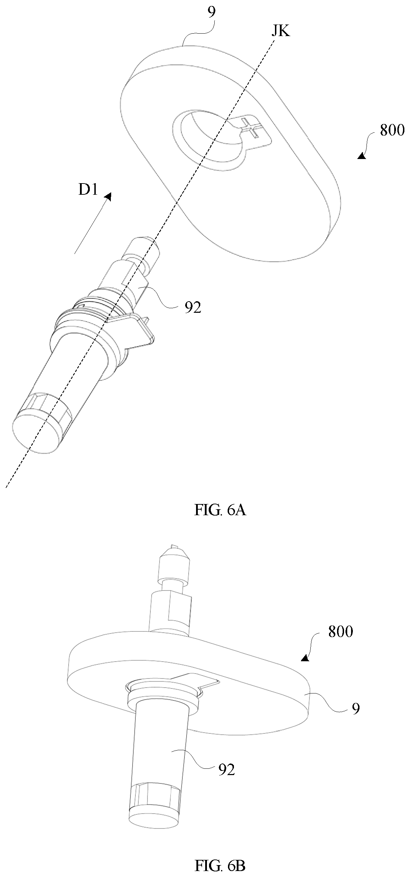

FIG. 4 is a schematic view illustrating a sterilization assembly 92 according to an example of the present disclosure. FIG. 5 is an exploded view of the sterilization assembly 92 according to an example of the present disclosure. FIG. 6A is a schematic view illustrating the sterilization assembly 92 and the electronic assembly 9 before assembly according to an example of the present disclosure. FIG. 6B is a schematic view illustrating the sterilization assembly 92 and the electronic assembly 9 after assembly according to an example of the present disclosure. In FIGS. 5 and 6A, line JK schematically represents a central axis of the sterilization assembly 92.

In some examples, the sensor 8 may first be sealed, the sterilization assembly 92 containing the sensor 8 may then be sterilized, and thereafter the sterilization assembly 92 may be fitted to the electronic assembly 9 and further assembled with the applicator device 100. In such a case, compared with conventional arrangements in which the sensor 8 and the electronic assembly 9 are sterilized separately and a user must, prior to use, pick up the sensor 8 from another container and assemble it with the electronic assembly 9 using an applicator device containing the electronic assembly 9 in order to form a complete monitoring device 800, the applicator device 100 of the present disclosure is capable of accommodating a complete monitoring device 800. Thus, no additional pick-and-assembly operation is required before use, thereby facilitating use by the operator.

In the present disclosure, various approaches may be employed for sealing the sensor 8. One embodiment of sealing the sensor 8 is described below; however, the specific structure is not limited thereto.

In some examples, the monitoring device 800 may comprise a sealing assembly 93 (see FIG. 4). The sealing assembly 93 may be configured to seal the sensor 8. More specifically, the sealing assembly 93 may seal the implanted portion 81 of the sensor 8. In this case, the sealing assembly 93 that seals the sensor 8 helps maintain the sterile condition of the sensor 8 over an extended period of time, thereby extending the standby life of the monitoring device 800 (the standby life referring to the time period from completion of sterilization and factory assembly of the monitoring device 800 to its use by an operator, and also referred to as shelf life).

In some examples, the sensor 8 may be sterilized and retained within the sealing assembly 93, after which the sensor 8 and the sealing assembly 93 may be fitted onto the electronic assembly 9 to form the monitoring device 800, and the monitoring device 800 may then be mounted to the applicator device 100. In other words, the sealing assembly 93 may form part of the sterilization assembly 92. Before the monitoring device 800 is applied to a host using the applicator device 100, the sealing assembly 93 may be removed to place the monitoring device 800 into a ready-to-apply state. In such a case, compared with conventional arrangements in which the sensor 8 and the electronic assembly 9 are separately packaged and sterilized and a user must retrieve the sensor 8 from another container and assemble it with the electronic assembly 9 to form a complete monitoring device 800 prior to use, the applicator device 100 of the present disclosure can accommodate a complete monitoring device 800. Accordingly, no additional pick-and-assembly operation is required before use, thereby facilitating use by the operator.

In some examples, the monitoring device 800 may comprise a mounting base 94 (see FIG. 4). The sensor 8 may be assembled with the electronic assembly 9 by means of the mounting base 94. In some examples, the mounting base 94 may comprise a first through-hole 941 (see FIG. 5). The first through-hole 941 may serve as a passage through which the sensor 8 extends outwardly. In some examples, one end of the sensor 8 may extend through a sidewall of the mounting base 94. More specifically, the connection portion 82 of the sensor 8 may extend through the sidewall of the mounting base 94. In some examples, the electronic assembly 9 may comprise an electrical connection seat. After the sensor 8 has been assembled with the electronic assembly 9 via the mounting base 94, the sensor 8 may be electrically connected to electronic devices within the electronic assembly 9 through the electrical connection seat.

In some examples, the sealing assembly 93 may cooperate with the mounting base 94 to seal the sensor 8. More specifically, the implanted portion 81 of the sensor 8 may extend outwardly from the mounting base 94, and the sealing assembly 93 may abut against the mounting base 94 and surround the implanted portion 81 extending from the mounting base 94 peripherally. In other words, the sealing assembly 93 may seal one end of the first through-hole 941.

In some examples, the applicator device 100 may comprise a penetrating member 40 (see FIGS. 4 and 5). The penetrating member 40 may be configured to introduce at least a portion of the monitoring device 800 into a subcutaneous region of a host. For example, the penetrating member 40 may introduce the implanted portion 81 of the sensor 8 into the subcutaneous region of a host. In some examples, the penetrating member 40 may comprise a needle portion 41 and a carrying portion 42 connected to the needle portion 41 (see FIG. 5). The needle portion 41 may accommodate the sensor 8. In some examples, the sealing assembly 93, the mounting base 94, and the penetrating member 40 may cooperate to seal the sensor 8 (the specific structure of the penetrating member 40 will be described in greater detail later, while the present discussion focuses primarily on sealing of the sensor 8). Specifically, the first through-hole 941 of the mounting base 94 may serve as a passage through which the penetrating member 40 extends downwardly. The carrying portion 42 of the penetrating member 40 may abut against an upper surface of the mounting base 94, thereby sealing an upper end of the first through-hole 941. The needle portion 41 may extend through the first through-hole 941 and extend downwardly while accommodating the implanted portion 81 of the sensor 8. The sealing assembly 93 may surround the needle portion 41 and the portion of the sensor 8 extending through the mounting base 94 peripherally. At this point, the penetrating member 40 and the sealing assembly 93 respectively seal opposite ends of the first through-hole 941, thereby sealing the sensor 8 within a sealed space defined by the penetrating member 40, the mounting base 94, and the sealing assembly 93. In some examples, the carrying portion 42 may also extend downwardly through the first through-hole 941, and the sealing assembly 93 may be detachably connected to the carrying portion 42.

In some examples, the sterilization assembly 92 may further comprise sealing members. For example, the sterilization assembly 92 may comprise a first sealing member 951 and a second sealing member 952 (see FIG. 5). Here, the first sealing member 951 may be disposed between the carrying portion 42 and the upper surface of the mounting base 94, thereby improving the sealing performance of the sterilization assembly 92. The second sealing member 952 may be disposed between the sealing assembly 93 and a lower surface of the mounting base 94, thereby further improving the sealing performance of the sterilization assembly 92.

In some examples, the sterilization assembly 92, including the mounting base 94, the sealing assembly 93, the penetrating member 40, and the sensor 8, may be sterilized first and then fitted to the electronic assembly 9 before being assembled with the applicator device 100. Referring to FIG. 6A, the sterilization assembly 92 may be fitted to the electronic assembly 9 along direction D1, thereby forming an assembly containing the monitoring device 800 that can be mounted to the applicator device 100, as shown in FIG. 6B.

The present disclosure also contemplates other variations for sealing the sensor 8. For example, in some examples, the enclosure 91 of the electronic assembly 9 may comprise an upper enclosure 911 and a lower enclosure 912 (see FIG. 2). Electronic devices may be disposed within an accommodation space formed by the upper enclosure 911 and the lower enclosure 912. In some examples, the mounting base 94 may be the upper enclosure 911 or the lower enclosure 912 of the electronic assembly 9 and may cooperate with the sealing assembly 93 and the penetrating member 40 to seal the sensor 8. The specific structure may be obtained through appropriate modifications to the mounting base 94 described above and will not be repeated herein.

In some examples, the electronic assembly 9 may be adhered to the body surface of a host. For example, the monitoring device 800 may further comprise an adhesive pad disposed on a bottom surface of the enclosure 91 of the electronic assembly 9 and having adhesive properties. The electronic assembly 9 may thereby be adhered to the body surface of the host by means of the adhesive pad.

In some examples, the electronic assembly 9 may be configured to communicate with an external device via wireless communication modes or wired communication modes. In such a case, by configuring the electronic assembly 9 to communicate with an external device, analyte information acquired by the sensor 8 may be read in real time or at predetermined intervals. The external device may be a reader device 900.

In some examples, the monitoring device 800 may be communicatively connected to the reader device 900. In some examples, the reader device 900 may be a display device having communication functionality. In some examples, communication functionality of the reader device 900 may be achieved through wireless communication modes or wired communication modes. Wireless communication modes may comprise Bluetooth, NFC, Wi-Fi, and the like. Wired communication modes may comprise USB, optical fiber, and the like. In addition, the reader device 900 may be a smart terminal on which an application matched with the monitoring device 800 is installed. Examples of smart terminals include notebook computers, tablet computers, and smart phones.

As described above, the monitoring device 800 according to the present disclosure may be applied to a host by means of the applicator device 100. The applicator device 100 according to the present disclosure will now be described in detail with reference to the accompanying drawings.

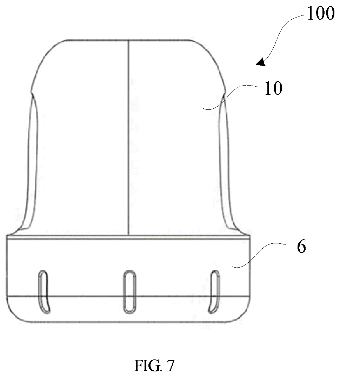

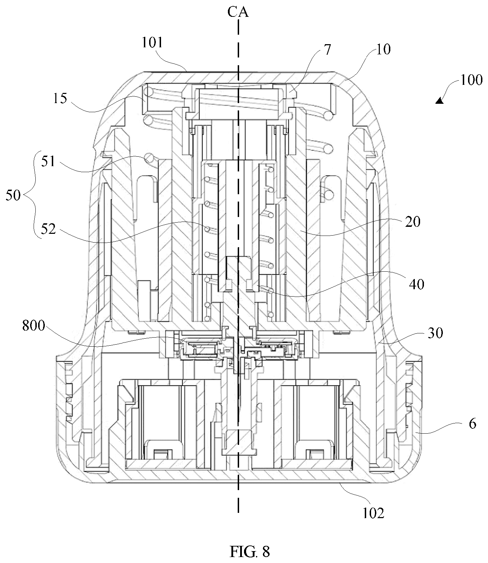

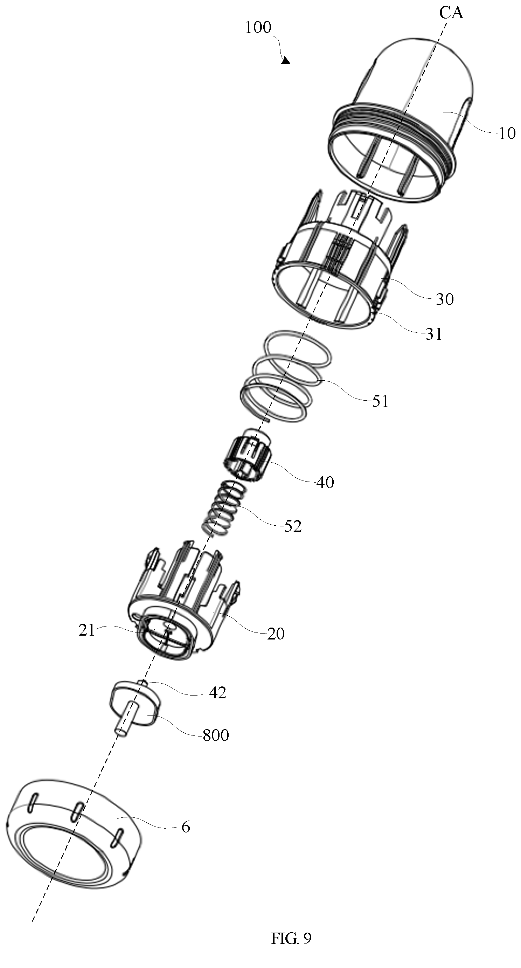

FIG. 7 is a schematic overall perspective view of the applicator device 100 according to an example of the present disclosure. FIG. 8 is a cross-sectional view of the applicator device 100 according to an example of the present disclosure. FIG. 9 is an exploded view of the applicator device 100 according to an example of the present disclosure. In FIGS. 8 and 9, line CA schematically represents a central axis CA of the applicator device 100. Hereinafter, the term "central axis CA" refers to the central axis CA of the applicator device 100.

In some examples, the applicator device 100 may comprise a first housing 10 configured to provide a movement space, and a movable body 20 (see FIGS. 7, 8, and 9). The movable body 20 may accommodate the monitoring device 800 and be configured to move within the movement space of the first housing 10. In some examples, the applicator device 100 may comprise a proximal end 101 positioned proximal to the host during operation and a distal end 102 positioned distal to the host during operation (see FIG. 8). As used herein, the term "proximal end 101" may be understood as an end positioned proximal to the host during operation, and the term "distal end 102" may be understood as an end positioned distal to the host during operation.

In some examples, the movable body 20 may be releasably retained by the first housing 10. When the movable body 20 is released, the movable body 20 may move within the movement space of the first housing 10.

In some examples, the applicator device 100 may comprise a second housing 30 (see FIG. 8 or FIG. 9). The second housing 30 may move relative to the first housing 10. In some examples, the second housing 30 may be at least partially disposed within an accommodation space of the first housing 10. In some examples, the first housing 10 and the second housing 30 may form a movement space after being fitted and combined. The movable body 20 may move within the movement space formed after the first housing 10 and the second housing 30 being fitted and combined.

In some examples, the second housing 30 may constrain a movement path of the movable body 20. That is, when the movable body 20 is released, it may move along the movement path defined by the second housing 30. In some examples, an axis of the first housing 10 and an axis of the second housing 30 may be substantially parallel to the central axis CA of the applicator device 100. In some examples, the first housing 10 and the second housing 30 may be arranged coaxially. For example, in FIG. 8, the axes of the first housing 10 and the second housing 30 may coincide with the central axis CA of the applicator device 100.

In some examples, the second housing 30 and the first housing 10 may be moved relative to each other to release the movable body 20. In this case, cooperation between the two housings can release the lock on the movable body 20, thereby facilitating use by an operator. In some examples, the second housing 30 may define an attachment location of the monitoring device 800 on a body surface of the host. In some examples, the second housing 30 may comprise a proximal surface 31 configured to contact the skin of the host during operation (see FIG. 9). In this case, by placing the proximal surface 31 of the second housing 30 against the skin of the host, on one hand, it becomes easier to apply force to cause relative movement between the first housing 10 and the second housing 30 (hereinafter referred to as relative movement of the two housings), thereby unlocking the movable body 20; and on the other hand, such arrangement also helps define the application site, thereby facilitating accurate application of the monitoring device 800 to the host. Furthermore, compared with applicator devices provided with a trigger button on the housing, the applicator device 100 of the present disclosure allows force to be applied through the proximal surface 31 of the second housing 30 over a larger contact area, and the linked triggering action of the two housings further improves ease of operation.

In some examples, the movable body 20 may comprise a receiving portion 21 (see FIG. 9). The receiving portion 21 may be configured to accommodate the monitoring device 800. In some examples, the monitoring device 800 may be releasably retained within the receiving portion 21. In this case, movement of the movable body 20 causes the monitoring device 800 to move together therewith, and after the monitoring device 800 has been applied to the host, the monitoring device 800 may be released from the receiving portion 21 and remain on the host for monitoring purposes.

In some examples, after being applied to the host, the monitoring device 800 may be released from retention by the movable body 20 and remain on the host. Accordingly, the monitoring device 800 applied to the host may monitor physiological parameters of the host. In some examples, the monitoring device 800 applied to the host may adhere to the body surface of the host. When the operator removes the applicator device 100 from the body surface of the host, the monitoring device 800 may remain adhered to the body surface of the host while being released from the receiving portion 21.

In some examples, as described above, the applicator device 100 may comprise a penetrating member 40 (see FIG. 8 or FIG. 9). The penetrating member 40 may introduce at least a portion of the monitoring device 800 into a subcutaneous region of the host. In some examples, the penetrating member 40 may be disposed on the movable body 20. When the movable body 20 is released and moves toward the proximal end 101, the penetrating member 40 disposed on the movable body 20 and the receiving portion 21 also move toward the proximal end 101, thereby applying the monitoring device 800 accommodated within the receiving portion 21 to the host. The penetrating member 40 may place at least a portion of the monitoring device 800 beneath the skin of the host.

In some examples, the penetrating member 40 may be releasably disposed on the movable body 20. When the penetrating member 40 is released, it may move relative to the movable body 20. For example, when released, the penetrating member 40 may move relative to the movable body 20 in a direction away from the host. Accordingly, the penetrating member 40 may be withdrawn from the host.

In some examples, the applicator device 100 may comprise a drive mechanism 50 (see FIG. 8). After the movable body 20 has been released, the movable body 20 and the penetrating member 40 may move together toward the proximal end 101 under the action of the drive mechanism 50 to apply the monitoring device 800 to the host, with at least a portion of the monitoring device 800 positioned beneath the skin of the host. In this case, the drive mechanism 50 can provide a relatively accurate driving force, thereby enabling more precise control of the penetrating force and penetrating depth of the penetrating member 40 during application and thereby facilitating accurate application of the monitoring device 800 to the host.

In some examples, the drive mechanism 50 may comprise a first drive portion 51 (see FIG. 9). The first drive portion 51 may be configured to drive the movable body 20. In some examples, the first drive portion 51 may apply an action to the movable body 20 in a direction toward the proximal end 101. When the movable body 20 is released, the movable body 20 may be driven by the first drive portion 51 toward the proximal end 101, thereby advancing the monitoring device 800 accommodated within the receiving portion 21 toward the host.

In other examples, the applicator device 100 may omit the first drive portion 51. In such cases, when the movable body 20 is released, the movable body 20 may be manually driven to move toward the proximal end 101.

In some examples, the drive mechanism 50 may comprise a second drive portion 52 (see FIG. 9). The second drive portion 52 may be configured to drive the penetrating member 40. In some examples, the second drive portion 52 may apply an action to the penetrating member 40 in a direction toward the distal end 102. When the penetrating member 40 is released, the penetrating member 40 may move toward the distal end 102 under the action of the second drive portion 52, thereby causing the penetrating member 40 to withdraw from the host. In this case, use of the second drive portion 52 to perform the retraction operation of the penetrating member 40 enables more precise control over the timing at which the penetrating member 40 withdraws from the host.

In other examples, the applicator device 100 may omit the second drive portion 52. In such cases, the penetrating member 40 may be manually driven to withdraw from the host. For example, in some examples, the penetrating member 40 may be integrally connected with the movable body 20, such that the penetrating member 40 is withdrawn from the host by manually moving the movable body 20 in a direction away from the host.

In some examples, the applicator device 100 may comprise a transmission mechanism 7 (see FIG. 8). In some examples, the transmission mechanism 7 may abut an end of the movable body 20 facing the distal end 102, and opposite ends of the first drive portion 51 may respectively abut the transmission mechanism 7 and the first housing 10. In such a case, the first drive portion 51 can apply an action toward the proximal end 101 to the movable body 20 through the transmission mechanism 7. In some examples, the transmission mechanism 7 may accommodate the penetrating member 40 after the penetrating member 40 has been released and moved toward the distal end 102. In this case, the transmission mechanism 7 helps maintain the penetrating member 40 in a stable position within the first housing 10 after movement toward the distal end 102.

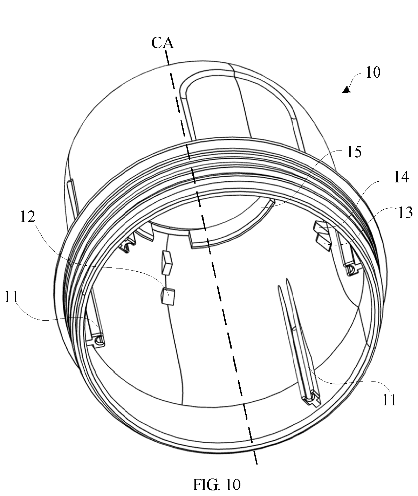

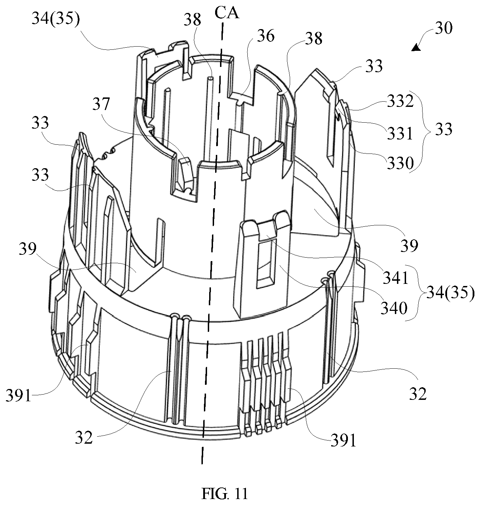

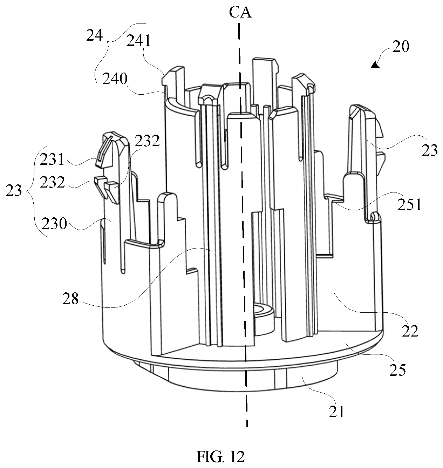

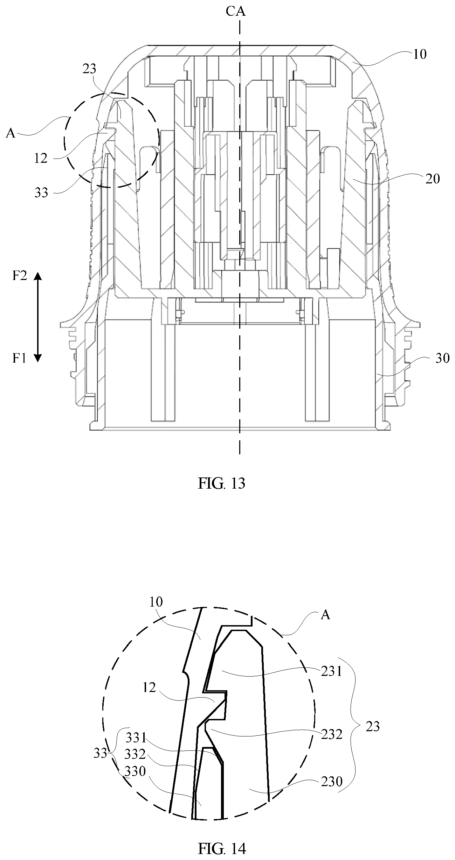

FIG. 10 is a schematic view of the first housing 10 according to an example of the present disclosure. FIG. 11 is a schematic view of the second housing 30 according to an example of the present disclosure. FIG. 12 is a schematic view of the movable body 20 according to an example of the present disclosure. FIG. 13 is a cross-sectional schematic view of the first housing 10, the second housing 30, and the movable body 20 after assembly.

In some examples, one or more rib-like protrusions 11 extending substantially along the direction of the central axis CA may be provided on an inner wall of the first housing 10 (see FIG. 10). In some examples, the rib-like protrusions 11 may function as reinforcing ribs to effectively improve resistance of the first housing 10 to deformation. In some examples, the rib-like protrusions 11 may additionally function as guide ribs to facilitate fitting of the first housing 10 and the second housing 30. Here, such guide ribs may further help suppress undesired rotation of the second housing 30 within the first housing 10.

In some examples, male and female mechanical fit structures may be provided on the inner wall of the first housing 10 and the outer wall of the second housing 30. In some examples, the male and female mechanical fit structures may comprise protrusion-and-groove fit structures. For example, in some examples, one or more rib-like protrusions 11 may be provided on the inner wall of the first housing 10, and one or more rib-like grooves 32 may be provided on the outer wall of the second housing 30 (see FIGS. 10 and 11). When the second housing 30 is fitted into the first housing 10, the one or more rib-like protrusions 11 of the first housing 10 may respectively fit within the one or more rib-like grooves 32 of the second housing 30. In this case, on one hand, matching of the rib-like protrusions 11 and the rib-like grooves 32 facilitates fitting of the first housing 10 and the second housing 30; and on the other hand, the rib-like protrusions 11 and the rib-like grooves 32 may further guide the direction of relative movement between the first housing 10 and the second housing 30, and cooperation of the rib-like protrusions 11 and the rib-like grooves 32 helps suppress undesired rotation of the second housing 30. In some examples, a plurality of rib-like protrusions 11 provided on the inner wall of the first housing 10 may be evenly or symmetrically distributed around the inner wall of the first housing 10.

In some examples, recessed structures may be formed in the rib-like protrusions 11 on the inner wall of the first housing 10, and corresponding protruding structures may be formed in the rib-like grooves 32 on the outer wall of the second housing 30. When the second housing 30 is fitted into the first housing 10, the rib-like protrusions 11 of the first housing 10 may be positioned within the rib-like grooves 32 of the second housing 30, and the protruding structures within the rib-like grooves 32 may be positioned within the recessed structures of the rib-like protrusions 11. Such an arrangement thus may further help improve fitting stability between the first housing 10 and the second housing 30 and may further define the guide path, thereby promoting more accurate directional guidance during relative movement between the first housing 10 and the second housing 30.

In some examples, as described above, the movable body 20 may be releasably retained by the first housing 10. In some examples, the first housing 10 may comprise a first retaining portion 12 (see FIG. 10). The first retaining portion 12 may be configured to releasably retain the movable body 20. In some examples, the first retaining portion 12 may be provided on the inner wall of the first housing 10. For example, the first retaining portion 12 may be a protruding structure formed on the inner wall of the first housing 10.

In some examples, the movable body 20 may comprise a first locking portion 23 (see FIG. 12). Here, the first locking portion 23 may be configured to releasably interlock with the first retaining portion 12. Accordingly, the movable body 20 may be releasably retained by the first retaining portion 12 through cooperation between the first locking portion 23 and the first retaining portion 12. In some examples, the movable body 20 may comprise a sidewall 22. In some examples, the first locking portion 23 may be provided on the sidewall 22 of the movable body 20.

In some examples, interlocking between the first retaining portion 12 and a retained component may be achieved by means of at least one structure of a latch, hook, catch, or pin. For example, in some examples, interlocking between the first retaining portion 12 and the first locking portion 23 may be achieved through at least one structure of a latch, hook, catch, or pin. In such a case, the first retaining portion 12 and the first locking portion 23 are releasably interlocked through at least one structure of a latch, hook, catch, or pin, thereby being capable of providing a convenient release manner.

In some examples, as described above, the first retaining portion 12 may be a protruding structure formed on the inner wall of the first housing 10 (see FIG. 10). In some examples, when the movable body 20 is retained by the first retaining portion 12, the first locking portion 23 engages the protruding structure. The first locking portion 23 may be separated from the first retaining portion 12 by deflecting the first locking portion 23 in a direction away from the first retaining portion 12. Because typically in order to cause a subject component to offset, generally this is done by adjusting its material or structure, such as by employing a relatively soft material to prepare the subject component or providing a subject component having a certain length, such a component can offset under an external force. In the present disclosure, however, the first housing 10 is generally prepared from a relatively rigid material so as to protect internal structures by the first housing 10. In such a case, compared with providing a first retaining portion 12 that may offset and have a certain length within the first housing 10, providing the first retaining portion 12 as a protruding structure formed on the inner wall of the first housing 10 can reduce complexity of preparing process of the first housing 10 while decreasing the space required for providing the first retaining portion 12, thereby facilitating miniaturization of the applicator device 100 and ease of use by an operator.

In some examples, the first retaining portion 12 may comprise a surface facing the distal end 102, and the first locking portion 23 may comprise a surface facing the proximal end 101. When the movable body 20 is retained, the distal-facing surface of the first retaining portion 12 and the proximal-facing surface of the first locking portion 23 may engage one another. In some examples, when the movable body 20 is released, the distal-facing surface of the first retaining portion 12 and the proximal-facing surface of the first locking portion 23 may be separated from one another.

In some examples, the first locking portion 23 may comprise an arm 230 extending substantially along the direction of the central axis CA (hereinafter referred to as a first arm 230 for ease of reference) and a protrusion 231 linked to the first arm 230 and protruding away from the central axis CA in a direction substantially orthogonal to the central axis CA (hereinafter referred to as a first protrusion 231 for ease of reference) (see FIG. 12). In some examples, the first arm 230 may be formed on the sidewall 22 of the movable body 20. In such a case, when the first protrusion 231 of the first locking portion 23 is engaged to the first retaining portion 12, actuation of the first protrusion 231 toward the central axis CA causes the first protrusion 231 to disengage from the first retaining portion 12 (that is, the first locking portion 23 is separated from the first retaining portion 12), thereby conveniently releasing the first locking portion 23 from the first retaining portion 12.

In some examples, the first arm 230 may be elastic. In some examples, the first arm 230 may exhibit elasticity in a direction substantially orthogonal to the central axis CA. In some examples, the first arm 230 may tend to expand away from the central axis CA in a direction extending from the proximal end 101 toward the distal end 102. In other words, in a natural state (i.e., in a state without an external force), the end of the first arm 230 proximal to the proximal end 101 is located closer to the central axis CA than the end of the first arm 230 proximal to the distal end 102. In other examples, the first arm 230 may be substantially parallel to the central axis CA. In some examples, when a force is applied to the first arm 230 in a direction substantially orthogonal to the central axis CA, the first arm 230 may pivot. For example, when a force directed toward the central axis CA is applied to the first arm 230 in a direction substantially orthogonal to the central axis CA, the first arm 230 may deflect such that the end thereof proximal to the distal end 102 moves toward the central axis CA. Accordingly, the movable body 20 can be released in a simple and convenient operation.

In some examples, the first arm 230 may expand away from the central axis CA in the direction extending from the proximal end 101 toward the distal end 102, and the first protrusion 231 of the first locking portion 23 may protrude away from the central axis CA. In such a case, the first locking portion 23 as a whole may expand outwardly to interlock with the first retaining portion 12 on an outer side (away from the central axis CA), and actuation of the first locking portion 23 inwardly (toward the central axis CA) may disengage the first locking portion 23 from the first retaining portion 12, thereby releasing the first locking portion 23.

In some examples, the first protrusion 231 may comprise a surface facing the proximal end 101. In some examples, the proximal-facing surface of the first protrusion 231 may be substantially orthogonal to the central axis CA. The first locking portion 23 may be retained by engaging or abutting against a distal-facing surface of the first retaining portion 12. Such an arrangement helps prevent movement of the movable body 20 toward the proximal end 101. When the first locking portion 23 is actuated such that the proximal-facing surface of the first protrusion 231 moves away from its original position, i.e., no longer engages the distal-facing surface of the first retaining portion 12, the first locking portion 23 is released. In other words, the first locking portion 23 may be a latch, hook, catch, pin, or similar structure formed on the movable body 20. For example, the first locking portion 23 may be formed as a finger-shaped structure, a straight structure, an L-shaped structure, a J-shaped structure, a Z-shaped structure, or another structure that may be conceptually divided into at least two portions, wherein at least one portion thereof (for example, the first arm 230 connected to the sidewall 22 of the movable body 20) may pivot, and another portion thereof (for example, a protruding structure that projects outwardly from the first arm 230 and abuts against the first retaining portion 12) may move away from its initial position with the pivoting motion, thereby releasing the retained component from being hooked, hung, latched, propped, supported, or the like.

In some examples, the number of first retaining portions 12 may be one or more. For example, the number of first retaining portions 12 may be one, two, three, or four. Correspondingly, the number of first locking portions 23 may be the same as the number of first retaining portions 12. In the embodiment shown in FIG. 12, two first locking portions 23 are provided, and the two first locking portions 23 may be symmetrically arranged on opposite sides of the movable body 20. Such an arrangement facilitates stable retention of the movable body 20 and improves the reliability of the applicator device 100.

In some examples, as described above, the first housing 10 and the second housing 30 may move relative to each other to release the movable body 20. In some examples, the second housing 30 may comprise a first trigger portion 33. The first trigger portion 33 may be configured to separate the first retaining portion 12 from the first locking portion 23 so as to release the movable body 20. Accordingly, the first trigger portion 33 facilitates release of the movable body 20 through relative movement of the two housings.

In some examples, the second housing 30 and the first housing 10 may move relative to each other toward one another to release the movable body 20 from retention by the first retaining portion 12. In some examples, the first housing 10 and the second housing 30 may move relative to each other along the direction of the central axis CA to release the movable body 20 from retention by the first retaining portion 12. In such a case, simple relative movement of the two housings along the direction of the central axis CA enables the first trigger portion 33 to separate the first locking portion 23 from the first retaining portion 12, thereby facilitating use by an operator. In the example illustrated in FIG. 13, relative movement of the first housing 10 and the second housing 30 toward one another may comprise movement of the first housing 10 in direction F1 (in a direction toward the proximal end 101), movement of the second housing 30 in direction F2 (in a direction toward the distal end 102), or simultaneous movement of the first housing 10 in direction F1 and the second housing 30 in direction F2.

In some examples, the first trigger portion 33 may be formed on a sidewall of the second housing 30. In some examples, the first trigger portion 33 may comprise a trigger arm 330 extending substantially along the direction of the central axis CA (see FIG. 11). When the first housing 10 and the second housing 30 move relative to each other, the trigger arm 330 may act upon the first locking portion 23 to separate the first locking portion 23 from the first retaining portion 12.

In some examples, the first locking portion 23 may further comprise a protrusion 232 extending away from the central axis CA in a direction substantially orthogonal to the central axis CA (hereinafter referred to as a second protrusion 232 for ease of reference) (see FIG. 12). The second protrusion 232 may be provided on the first arm 230. In some examples, the first trigger portion 33 may apply a force to the second protrusion 232 so as to cause the first arm 230 to offset.

In some examples, the second protrusion 232 may comprise a surface facing the proximal end 101. In some examples, the trigger arm 330 may comprise a surface facing the distal end 102. When the first housing 10 and the second housing 30 move relative to each other toward one another, the first trigger portion 33 first moves into abutting with the second protrusion 232 such that the distal-facing surface of the trigger arm 330 contacts the proximal-facing surface of the second protrusion 232. In the course of the continued relative movement, the second protrusion 232 is compressed, causing the first arm 230 gradually to deflect in a direction toward the central axis CA. When the first arm 230 has deflected to disengage from the first retaining portion 12, the movable body 20 is in a state of being released. Accordingly, the movable body 20 can be conveniently unlocked through relative movement of the first housing 10 and the second housing 30, thereby facilitating use by an operator.

In some examples, the surface facing the proximal end 101 of the second protrusion 232 and/or the surface facing the distal end 102 of the trigger arm 330 may be an inclined surface or a curved surface. In such a case, this arrangement facilitates movement of the second protrusion 232 under the action of the trigger arm 330 when the second protrusion 232 abuts the trigger arm 330.

In some examples, when the movable body 20 is retained by the first retaining portion 12, the second protrusion 232 may be positioned closer to the first trigger portion 33 than the first protrusion 231. In other words, when the movable body 20 is retained by the first retaining portion 12, the second protrusion 232 is located closer to the proximal end 101 than the first protrusion 231 in the direction extending from the proximal end 101 toward the distal end 102. In such a case, this arrangement facilitates unlocking of the movable body 20 through relative movement of the first housing 10 and the second housing 30 toward one another.

In some examples, the first protrusion 231 and the second protrusion 232 may be arranged as misaligned from one another in a vertical direction and/or a horizontal direction. Here, a misaligned arrangement in the vertical direction may mean that the axes in the vertical direction of the first protrusion 231 and the second protrusion 232 do not overlap, or that when being projected in the vertical direction, the first protrusion 231 and the second protrusion 232 do not overlap. A misaligned arrangement in the horizontal direction may be understood similarly. In such a case, because the first protrusion 231, the second protrusion 232, the first retaining portion 12 of the first housing 10, and the trigger arm 330 of the second housing 30 are associated with each other, design considerations comprise relative positioning of the components and efficient utilization of the internal space of the first housing 10. In the present embodiment, by configuring the first protrusion 231 and the second protrusion 232 to be misaligned in the vertical direction and/or the horizontal direction, compared with arranging them on a common axis, space can be conserved, integration of internal structures within the applicator device 100 can be improved, and miniaturization of the applicator device 100 can be facilitated.

In some examples, the first protrusion 231 and the second protrusion 232 may be misaligned from one another in both the vertical and horizontal directions. When the movable body 20 is retained by the first retaining portion 12, the first retaining portion 12 may be horizontally adjacent to the second protrusion 232 and in contact with the first protrusion 231, while the first retaining portion 12 may overlap the first protrusion 231 in the vertical direction. In such a case, after the first protrusion 231 is actuated and disengages from the first retaining portion 12, the second protrusion 232 and the trigger arm 330 are not located in the movement path of the first protrusion 231 toward the proximal end 101, thereby facilitating smooth actuation of the movable body 20 toward the host.

In some examples, a single first locking portion 23 may comprise one or more second protrusions 232. For example, the number of second protrusions 232 may be one, two, three, or four. In the embodiment shown in FIG. 12, four second protrusions 232 are provided (one of which is not visible due to the viewing angle), with the four second protrusions 232 arranged in pairs on the first locking portion 23. Correspondingly, the number of first trigger portions 33 may be the same as the number of second protrusions 232. In the embodiment shown in FIG. 11, four first trigger portions 33 are provided, and the four first trigger portions 33 correspond in position to the four second protrusions 232. In such a case, this arrangement facilitates application of force from the first trigger portions 33 to the second protrusions 232 during relative movement of the two housings, thereby separating the first locking portion 23 from the first retaining portion 12.

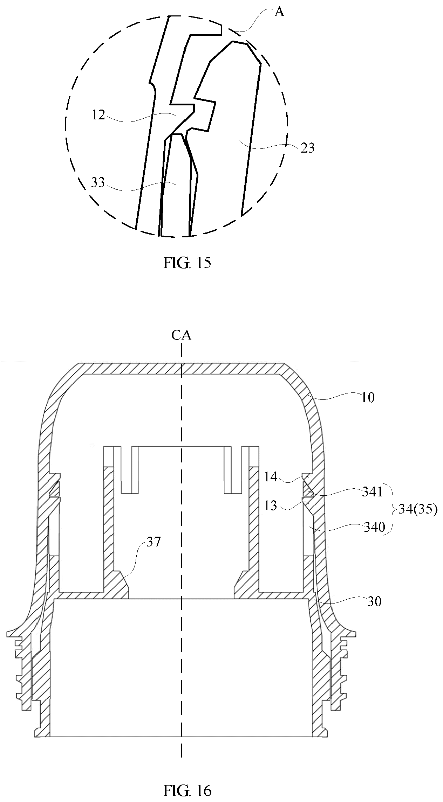

FIG. 14 is a cross-sectional schematic view illustrating the first locking portion 23 before actuation by the first trigger portion 33 according to an example of the present disclosure. FIG. 15 is a cross-sectional schematic view illustrating the first locking portion 23 after actuation by the first trigger portion 33 according to an example of the present disclosure. It should be noted that FIGS. 14 and 15 are enlarged views of region A in FIG. 13. FIGS. 14 and 15 principally illustrate triggering of the first locking portion 23 by the first trigger portion 33. For clarity, certain lines that could obscure the illustration have been omitted, and gaps between components have been exaggerated. In addition, for ease of illustration, only the first retaining portion 12, the first locking portion 23, and the first trigger portion 33 are labeled in FIG. 15. Structural details of these components may be understood with reference to FIG. 14.

The triggering of the first trigger portion 33 on the first locking portion 23 will now be described in detail with reference to FIGS. 14 and 15. As described above, prior to actuation of the first locking portion 23 by the first trigger portion 33, the first locking portion 23 is in contact with the first retaining portion 12, and the first retaining portion 12 restrains movement of the first locking portion 23 toward the proximal end 101, thereby retaining the movable body 20 (see FIG. 14). When the first trigger portion 33 actuates the first locking portion 23 (for example, when the two housings move relative to each other toward one another), the first arm 230 and the first protrusion 231 are gradually gathered inwardly under the action of the first trigger portion 33 (i.e., moved in a direction toward the central axis CA). When the first protrusion 231 is moved to a position at which it is separated from the first retaining portion 12, the movable body 20 is released (see FIG. 15).

In some examples, after the movable body 20 is released, the movable body 20 may move toward the proximal end 101. In some examples, when the movable body 20 applies the monitoring device 800 to the host, an end of the movable body 20 proximal to the proximal end 101 may be flush with the proximal end 101 of the applicator device 100. Accordingly, the monitoring device 800 accommodated within the receiving portion 21 can be conveniently applied to the host.

In some examples, the first retaining portion 12 comprises a surface facing the proximal end 101. In some examples, the first trigger portion 33 comprises a first surface 331 and a second surface 332, both facing the distal end 102 (see FIG. 14). Here, the first surface 331 may face the first locking portion 23, and the second surface 332 may face the first retaining portion 12. When the first housing 10 and the second housing 30 move relative to each other toward one another, the first surface 331 may first come into contact with the first locking portion 23 and apply a force to the first locking portion 23 in the course of travelling. In the course of continued travelling, the second surface 332 may contact the surface facing the proximal end 101 of the first retaining portion 12. Accordingly, a force directed toward the central axis CA can be conveniently applied to the first locking portion 23 so as to separate the first locking portion 23 from the first retaining portion 12.

In some examples, when the first housing 10 and the second housing 30 move relative to one another along the direction of the central axis CA, the first trigger portion 33 may act on the first locking portion 23 so as to separate the first locking portion 23 from the first retaining portion 12. In this case, simply moving the two housings relative to one another along the direction of the central axis CA enables the first trigger portion 33 to separate the first locking portion 23 from the first retaining portion 12, thereby facilitating use by an operator.

In some examples, the proximal surface 31 of the second housing 30 may be placed against the skin of the host, and an action may be applied to the first housing 10 toward the host (for example, an operator pressing the first housing 10 toward the host), thereby causing the first housing 10 and the second housing 30 to move relative to each other toward one another, thereby releasing the movable body 20. After being released, the movable body 20 and the penetrating member 40 move together toward the proximal end 101 to apply the monitoring device 800 to the body surface of the host, and the penetrating member 40 introduces at least a portion of the monitoring device 800 into a subcutaneous region of the host. Accordingly, the applicator device 100 may be conveniently operated with a single hand by the host.

In some examples, the second housing 30 may comprise one or more protruding structures 391 (see FIG. 11). In some examples, the protruding structures 391 may protrude outwardly from an inner wall of the second housing 30 in a direction substantially orthogonal to the central axis CA and away from the central axis CA. In some examples, the protruding structures 391 may have a clearance fit with the inner wall of the first housing 10. In such a case, the protruding structures 391 help suppress undesired wobbling and/or rotation of the second housing 30 within the first housing 10, thereby improving the reliability of the applicator device 100. In some examples, a plurality of protruding structures 391 may be provided as distributed around the outer periphery of the second housing 30.

In some examples, the first housing 10 may comprise a first restricting portion 13 (see FIG. 10). The first restricting portion 13 may impose restriction on the second housing 30 and inhibit the second housing 30 from disengaging from the first housing 10. Such an arrangement helps improve the reliability of the applicator device 100. That is, in some examples, the first housing 10 may comprise a first restricting portion 13 configured to inhibit separation of the second housing 30 from the first housing 10.

In some examples, the first restricting portion 13 may comprise a protruding structure formed on the inner wall of the first housing 10 (see FIG. 10).

In some examples, the second housing 30 may comprise a first restricted portion 34 (see FIG. 11). The first restricted portion 34 may be coupled to the first restricting portion 13. In some examples, the first restricted portion 34 may be disposed on a sidewall of the second housing 30. In some examples, interlocking between the first restricting portion 13 and the first restricted portion 34 may be achieved through at least one structure of a latch, hook, catch, or pin.

In some examples, when the movable body 20 is retained by the first retaining portion 12, the first restricted portion 34 may be coupled with the first restricting portion 13. Accordingly, the second housing 30 may be restrained while the movable body 20 remains retained by the first retaining portion 12 in a standby state, thereby inhibiting the second housing 30 from disengaging from the first housing 10.

In some examples, the first restricting portion 13 may comprise a surface facing the distal end 102. In some examples, the first restricted portion 34 may comprise a surface facing the proximal end 101. When the movable body 20 is retained by the first retaining portion 12, the surface facing the distal end 102 of the first restricting portion 13 may engage the surface facing the proximal end 101 of the first restricted portion 34. In some examples, when the movable body 20 is retained by the first retaining portion 12, the first restricted portion 34 is positioned closer to the distal end 102 than the first restricting portion 13.

In some examples, the first restricted portion 34 may comprise an extension portion 340 extending substantially along the direction of the central axis CA and a catch portion 341 linked with the extension portion 340 (see FIG. 11). When the catch portion 341 engages the first restricting portion 13, the second housing 30 is restrained to the first housing 10.

In some examples, the first housing 10 may comprise a second restricting portion 14 (see FIG. 10). The second restricting portion 14 may impose a restriction on the second housing 30. More specifically, the second restricting portion 14 may be configured to inhibit movement of the second housing 30 toward the proximal end 101. After the movable body 20 has been released as a result of relative movement between the first housing 10 and the second housing 30, the second housing 30 may be restrained by the second restricting portion 14. In this case, the second restricting portion 14 may restrain movement of the second housing 30 toward the proximal end 101, thereby preventing repeated use of the applicator device 100 and reducing the risk that a previously used penetrating member 40 that has not been re-sterilized may contact a host again. In some examples, the second restricting portion 14 may comprise a protruding structure formed on the inner wall of the first housing 10.

In some examples, the second housing 30 may comprise a second restricted portion 35 (see FIG. 11). The second restricted portion 35 may be configured to interlock with the second restricting portion 14. Accordingly, the second housing 30 may be retained by first retaining portion 12 through cooperation between the second restricted portion 35 and the second restricting portion 14. More specifically, after relative movement of the first housing 10 and the second housing 30 releases the movable body 20 from retention by the first retaining portion 12, the second restricted portion 35 may engage the second restricting portion 14.

In some examples, when the movable body 20 is retained by the first retaining portion 12, the second restricted portion 35 is positioned closer to the proximal end 101 than the second restricting portion 14; when the movable body 20 is in a released state, the second restricted portion 35 is positioned closer to the distal end 102 than the second restricting portion 14. In some examples, interlocking between the second restricting portion 14 and the second restricted portion 35 may be achieved through at least one structure of a latch, hook, catch, or pin.

In some examples, the second restricting portion 14 may comprise a surface facing the distal end 102. In some examples, the second restricted portion 35 may comprise a surface facing the proximal end 101. When the second housing 30 is retained, the surface facing the distal end 102 of the second restricting portion 14 and the surface facing the proximal end 101 of the second restricted portion 35 may engage with one another.

In some examples, the structure of the second restricted portion 35 may be identical to the structure of the first restricted portion 34. That is, the second restricted portion 35 may also comprise the extension portion 340 and the catch portion 341. In some examples, the first restricted portion 34 and the second restricted portion 35 may be the same structure. In other words, besides cooperating with the first restricting portion 13, the first restricted portion 34 may also cooperate with the second restricting portion 14 to retain the second housing 30. Stated differently, in some examples, the first restricted portion 34 may be configured to cooperate with the second restricting portion 14 to retain the second housing 30. Such an arrangement helps simplify the structure of the applicator device 100.

In some examples, the first restricted portion 34 may be formed as a structure similar to an H-shape or an arch shape (see FIG. 11). In such a case, the second housing 30 may be restrained by the first restricting portion 13 or the second restricting portion 14 by engaging a crossbar structure of the first restricted portion 34 with the first restricting portion 13 or the second restricting portion 14. In some examples, the extension portion 340 and the catch portion 341 may cooperate to form a similarly H-shaped structure or an arch-shaped structure. Here, the extension portion 340 may constitute a vertical structure of the H-shaped structure or arch-shaped structure, and the catch portion 341 may constitute the crossbar structure of the H-shaped structure. In such a case, on the one hand, the cooperation of the vertical structure helps suppress undesired rotation of the second housing within the first housing; and on the other hand, compared with an L-shaped structure, the structure formed by the extension portion 340 and the catch portion 341 is a single-layer structure in the same thickness direction, whereas an L-shaped structure is a double-layer structure. Accordingly, use of the single-layer structure reduces the space required in a given width direction of the applicator device 100, thereby facilitating the formation of a compact structure in cooperation with other components and improving utilization of the internal space of the first housing. In other examples, the extension portion 340 and the catch portion 341 may alternatively cooperate to form an L-shaped, J-shaped, Z-shaped, or similar structure.

In other examples, the extension portion 340 may comprise an arm extending substantially along the direction of the central axis CA (hereinafter referred to as a second arm for ease of reference) (see FIG. 11). In some examples, the catch portion 341 may be disposed on the extension portion 340 (see FIG. 11). In such a case, this arrangement facilitates cooperation between the extension portion 340 and the catch portion 341 to form a structure capable of coupling with the first restricting portion 13 and the second restricting portion 14. In some examples, the catch portion 341 and the extension portion 340 may be integrally formed.

In some examples, the second arm may be elastic. In some examples, the second arm may exhibit elasticity in a direction substantially orthogonal to the central axis CA. In some examples, the second arm may tend to expand away from the central axis CA in the direction from the proximal end 101 toward the distal end 102. In other words, in a natural state (i.e., in a state without an external force), the end of the second arm proximal to the proximal end 101 is closer to the central axis CA than the end of the second arm proximal to the distal end 102. In other examples, the second arm may extend substantially parallel to the central axis CA.

In some examples, when an action is applied to the second arm in a direction substantially orthogonal to the central axis CA, the second arm may pivot. For example, when an action directed toward the central axis CA is applied to the second arm in a direction substantially orthogonal to the central axis CA, the second arm may deflect such that the end thereof proximal to the distal end 102 moves toward the central axis CA. In such a case, when the first housing 10 and the second housing 30 move relative to each other toward one another, the second arm may deflect so that the catch portion 341 passes over and engages onto the second restricting portion 14.

In some examples, the second arm may be formed on a sidewall of the second housing 30. In some examples, the second arm may constitute a portion of the sidewall of the second housing 30. When the catch portion 341 of the second restricted portion 35 engages the second restricting portion 14, the second housing 30 may be retained.

In some examples, the second arm may expand away from the central axis CA in the direction extending from the proximal end 101 toward the distal end 102, and the catch portion 341 may protrude in a direction away from the central axis CA. In such a case, outward expansion of the second restricted portion 35 as a whole enables interlocking with the second restricting portion 14 on an outer side (i.e., away from the central axis CA).

In some examples, the second restricting portion 14 may comprise a surface facing the proximal end 101. In some examples, the catch portion 341 may comprise a surface facing the distal end 102. When the first housing 10 and the second housing 30 move relative to each other toward one another, the surface facing the distal end 102 of the catch portion 341 first contacts the surface facing the proximal end 101 of the second restricting portion 14. In the course of continued movement, the catch portion 341 is compressed by the second restricting portion 14 and deflects as a whole in a direction toward the central axis CA. When the catch portion 341 reaches a position in which the surface facing the proximal end 101 of the catch portion 341 is farther toward the distal end 102 than the surface facing the distal end 102 of the second restricting portion 14, the catch portion 341 is released from compression by the second restricting portion 14 and expands outwardly. As a result, the surface facing the distal end 102 of the second restricting portion 14 engages the surface facing the proximal end 101 of the catch portion 341, thereby retaining the second housing 30.

In some examples, the surface facing the proximal end 101 of the second restricting portion 14 and/or the surface facing the distal end 102 of the catch portion 341 may be an inclined surface or a curved surface. In such a case, this arrangement facilitates the catch portion 341 smoothly passing over the proximal-facing surface of the second restricting portion 14 and engaging the second restricting portion 14 while being compressed thereagainst as the two housings move relative to one another.

In some examples, after the movable body 20 has been released, the first restricted portion 34 may disengage from the first restricting portion 13. In some examples, after the movable body 20 has been released, the second housing 30 may be retained by the second restricting portion 14 via the second restricted portion 35.

In some examples, the structure of the first restricting portion 13 may be identical to that of the second restricting portion 14. In some examples, the first restricting portion 13 and the second restricting portion 14 may be arranged adjacent to one another along the direction of the central axis CA. In some examples, the first restricting portion 13 may be positioned closer to the proximal end 101 than the second restricting portion 14. In such a case, prior to release of the movable body 20, the second housing 30 may be restrained by the first housing 10 via cooperation between the first restricted portion 34 and the first restricting portion 13. After the first housing 10 and the second housing 30 move relative to each other toward one another and thereby release the movable body 20, the second housing 30 is restrained by the first housing 10 via cooperation between the first restricted portion 34 and the second restricting portion 14.

In some examples, the number of first restricting portions 13 may be one or more. For example, one, two, three, or four first restricting portions 13 may be provided. The number of first restricted portions 34 may correspond to the number of first restricting portions 13. In some examples, the number of second restricting portions 14 may likewise be one or more. For example, one, two, three, or four second restricting portions 14 may be provided.

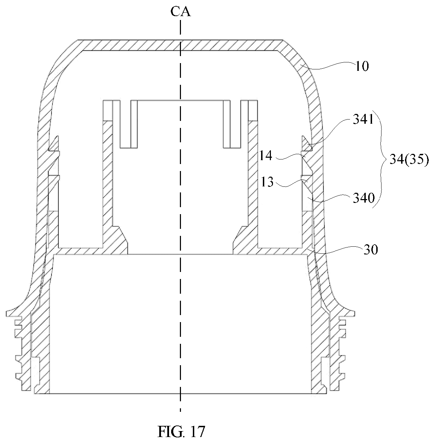

FIG. 16 is a cross-sectional schematic view illustrating the first restricted portion 34 not engaged with the second restricting portion 14 according to an example of the present disclosure. FIG. 17 is a cross-sectional schematic view illustrating the first restricted portion 34 engaged with the second restricting portion 14 according to an example of the present disclosure. It should be noted that FIGS. 16 and 17 primarily illustrate the cooperation among the first restricted portion 34, the first restricting portion 13, and the second restricting portion 14, and certain lines that might obscure or confuse the illustration have been omitted.

The cooperation among the first restricted portion 34, the first restricting portion 13, and the second restricting portion 14 will now be described in detail with reference to FIGS. 16 and 17. As described above, before the first trigger portion 33 actuates the first locking portion 23 (i.e., when the movable body 20 is retained by the first retaining portion 12 in a standby state), the first restricted portion 34 may engage the first restricting portion 13, and the first restricting portion 13 restrains movement of the second housing 30 toward the proximal end 101 (see FIG. 16). When the two housings are moved relative to each other toward one another along the direction of the central axis CA, the first restricted portion 34 is compressed inwardly by the second restricting portion 14, causing the second arm and the catch portion 341 gradually to gather inwardly (i.e., in a direction toward the central axis CA). When the surface facing the proximal end 101 of the catch portion 341 reaches a position beyond the surface facing the distal end 102 of the second restricting portion 14 toward the distal end 102, the catch portion 341 is released from compression by the second restricting portion 14 and expands outwardly. Consequently, the surface facing the distal end 102 of the second restricting portion 14 engages the surface facing the proximal end 101 of the catch portion 341, thereby retaining the second housing 30 (see FIG. 17).

In some examples, the second restricting portion 14 may not be provided. In such a case, when the force causing relative movement between the first housing 10 and the second housing 30 is stopped from imposing, the second housing 30 and the first housing 10 may automatically return to a standby position for subsequent use. In an example of a reusable applicator device 100, the needle portion 41 and the support seat 43 of the penetrating member 40 may be configured for detachable engagement. After a single application operation has been completed, the needle portion 41 may be detached from the support seat 43, replaced with a new sterilized needle portion 41, and a new monitoring device 800 may be fitted into the applicator device 100 for subsequent use.

In some examples, male and female mechanical fit structures may be provided on the sidewall of the second housing 30 and the sidewall 22 of the movable body 20. In some examples, the second housing 30 may comprise a third engagement feature 38, and the movable body 20 may comprise a fourth engagement feature 28 (see FIGS. 11 and 12). Cooperation between the third engagement feature 38 and the fourth engagement feature 28 may form the male and female mechanical fit structures. In some examples, the male and female mechanical fit structures may be a protrusion-and-groove cooperation structure. For example, the third engagement feature 38 may be one or more rib-like protrusions disposed on the sidewall of the second housing 30, and the fourth engagement feature 28 may be one or more rib-like grooves disposed on the sidewall 22 of the movable body 20 (see FIGS. 11 and 12). In some examples, when the movable body 20 is fitted into the second housing 30, the one or more rib-like protrusions of the second housing 30 may respectively fit into the one or more rib-like grooves of the movable body 20. In such a case, on one hand, this arrangement facilitates fitting of the second housing 30 and the movable body 20; and on the other hand, may guide the direction of movement of the movable body 20. Furthermore, this arrangement helps suppress undesired rotation of the movable body 20 through the cooperation between the rib-like protrusions and the rib-like grooves.

In some examples, a plurality of rib-like protrusions disposed on the sidewall of the second housing 30 may be uniformly or symmetrically distributed on the sidewall of the second housing 30.

In some examples, the second housing 30 may comprise a third restricting portion 36 (see FIG. 11). The third restricting portion 36 may impose a restriction on the movable body 20. In some examples, the third restricting portion 36 may be located at least partially in a travel path of the movable body 20 toward the proximal end 101. In such a case, when the movable body 20 moves toward the proximal end 101 and reaches the third restricting portion 36, the third restricting portion 36 may further restrict movement of the movable body 20, to stop the movement of the movable body 20. On the one hand, this enables the monitoring device 800 to be applied at a predetermined position; on the other hand, it prevents the movable body 20 from disengaging from the second housing 30. Accordingly, the reliability of the applicator device 100 is improved.

In some examples, the movable body 20 may comprise a third restricted portion 24 (see FIG. 12). The third restricted portion 24 may be coupled to the third restricting portion 36 so as to retain the movable body 20 to the second housing 30. In some examples, interlocking between the third restricting portion 36 and the third restricted portion 24 may be achieved through at least one structure of a latch, hook, catch, or pin. In some examples, the third restricting portion 36 may be disposed on a sidewall of the second housing 30, and the third restricted portion 24 may be disposed on the sidewall 22 of the movable body 20.

In some examples, when the movable body 20 is retained by the first retaining portion 12, the third restricted portion 24 may be positioned closer to the distal end 102 than the third restricting portion 36. In some examples, the third restricting portion 36 may comprise a surface facing the distal end 102. In some examples, the third restricted portion 24 may comprise a surface facing the proximal end 101. When the movable body 20 moves toward the proximal end 101 such that the third restricted portion 24 contacts the third restricting portion 36, the surface facing the distal end 102 of the third restricting portion 36 may engage the surface facing the proximal end 101 of the third restricted portion 24.

In some examples, the third restricted portion 24 may comprise an arm 240 extending substantially along the direction of the central axis CA (hereinafter referred to as a third arm 240 for ease of reference), and a protrusion 241 linked with the third arm 240 and protruding away from the central axis CA in a direction substantially orthogonal to the central axis CA (hereinafter referred to as a third protrusion 241 for ease of reference). In such a case, when the third protrusion 241 of the third restricted portion 24 engages the third restricting portion 36, movement of the movable body 20 may be restricted.

In some examples, the third arm 240 may be elastic. In some examples, the third arm 240 may exhibit elasticity in a direction substantially orthogonal to the central axis CA. In some examples, when an action is applied to the third arm 240 in a direction substantially orthogonal to the central axis CA, the third arm 240 may pivot. In such a case, this arrangement facilitates fitting of the movable body 20 into the second housing 30. In some examples, the third arm 240 may tend to expand away from the central axis CA in the direction extending from the proximal end 101 toward the distal end 102. In such a case, after the movable body 20 and the second housing 30 have been fitted into position, the third protrusion 241 may be positioned above the third restricting portion 36, enabling the third protrusion 241 to engage the third restricting portion 36 when the movable body 20 subsequently moves toward the proximal end 101.

In some examples, the axis of the second housing 30 and the axis of the movable body 20 may be substantially parallel to the central axis CA. In some examples, the second housing 30 and the movable body 20 may be arranged coaxially. For example, in FIG. 8, the axis of the second housing 30 and the axis of the movable body 20 may coincide with the central axis CA. In some examples, the second housing 30 and the movable body 20 may be arranged in a cross-overlap manner. More specifically, the second housing 30 may comprise a cutout portion 39 (see FIG. 11), through and into which certain members of the movable body 20 (for example, the first locking portion 23) may extend. In such a case, this arrangement increases utilization of the internal space of the first housing 10 and contributes to miniaturization of the applicator device 100. In some examples, the movable body 20 may be movably embedded within the second housing 30.

In some examples, the number of third restricting portions 36 may be one or more. For example, one, two, three, or four third restricting portions 36 may be provided. The number of third restricted portions 24 may correspond to the number of third restricting portions 36.

In some examples, the movable body 20 may comprise a sidewall and a first bottom portion 25 facing the proximal end 101 (see FIG. 12). In some examples, the first drive portion 51 may be positioned between the first housing 10 and the movable body 20. This arrangement facilitates application of an action from the first drive portion 51 to the movable body 20. For example, in some examples, the first drive portion 51 may be positioned between the first housing 10 and the first bottom portion 25 of the movable body 20. For example, in other examples, the first drive portion 51 may be positioned between the first housing 10 and a top portion of the movable body 20.

In some examples, a first positioning portion 251 for positioning the first drive portion 51 may be provided on the first bottom portion 25. In this case, the first positioning portion 251 may more stably define the location at which the first drive portion 51 applies an action to the movable body 20.

In some examples, the first positioning portion 251 of the first bottom portion 25 may be a stepped structure protruding from the first bottom portion 25 toward the distal end 102 along the central axis CA. The first drive portion 51 may be disposed within a groove-like space formed by the stepped structure and the sidewall of the second housing 30. In some examples, the first drive portion 51 may be disposed around the outer periphery of the sidewall 22 of the movable body 20 and abut against the first positioning portion 251.

In some examples, the first positioning portion 251 may have a cylindrical shape. In some examples, the first positioning portion 251 may be a solid cylindrical shape, and the first drive portion 51 may be disposed around the outer periphery of the first positioning portion 251. In other examples, the first positioning portion 251 may have a hollow cylindrical shape, and the first drive portion 51 may be disposed either around the outer periphery thereof or nested within the inner periphery thereof.

In some examples, the first drive portion 51 may have an energy-storing state and an energy-releasing state. When the first drive portion 51 switches from the energy-storing state to the energy-releasing state, the first drive portion 51 may release stored energy and apply an action to the movable body 20 in a direction toward the proximal end 101. When the movable body 20 is retained, the first drive portion 51 resides between the movable body 20 and the first housing 10 in the energy-storing state. When the movable body 20 is released, the first drive portion 51 may switch from the energy-storing state to the energy-releasing state and apply an action to the movable body 20 toward the proximal end 101.

In some examples, the first drive portion 51 may have a compressed state and an expanded state. When the first drive portion 51 switches from the compressed state to the expanded state, the first drive portion 51 releases energy. In some examples, the first drive portion 51 may comprise an elastic element. In some examples, opposite ends of the first drive portion 51 may respectively abut against the first housing 10 and the first bottom portion 25. When the movable body 20 is retained by the first retaining portion 12, the first drive portion 51 may be in the compressed state. In such a case, when the movable body 20 is released, the first drive portion 51 in the compressed state automatically applies a force to the movable body 20, thereby achieving automatic advancement of the movable body 20 and facilitating use by an operator. In some examples, the first drive portion 51 may be a spring.

In some examples, one end of the first drive portion 51 may be disposed around the first positioning portion 251 of the first bottom portion 25. In some examples, the first housing 10 may comprise a second positioning portion 15 (see FIG. 10). In some examples, the second positioning portion 15 may have a cylindrical shape. In such a case, the other end of the first drive portion 51 may be disposed around the second positioning portion 15. In some examples, the second positioning portion 15 may have a hollow cylindrical shape. In such a case, the other end of the first drive portion 51 may be disposed around or nested within the second positioning portion 15. In some examples, the second positioning portion 15 may be formed by at least two arcuate column segments. In such a case, the other end of the first drive portion 51 may be inserted between adjacent arcuate column segments.

In addition, the applicator device 100 according to the present embodiment is not limited thereto. In some examples, the applicator device 100 may exclude the first drive portion 51. In such a case, when the movable body 20 is released, the movable body 20 may also be moved toward the proximal end 101 by manually applying an action thereto.

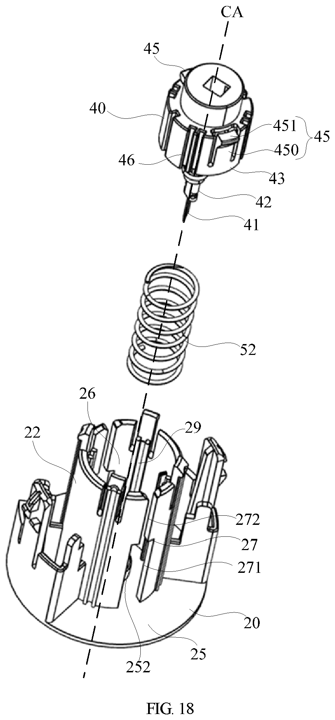

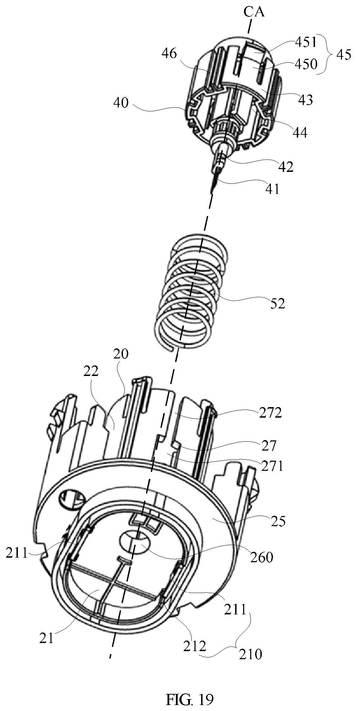

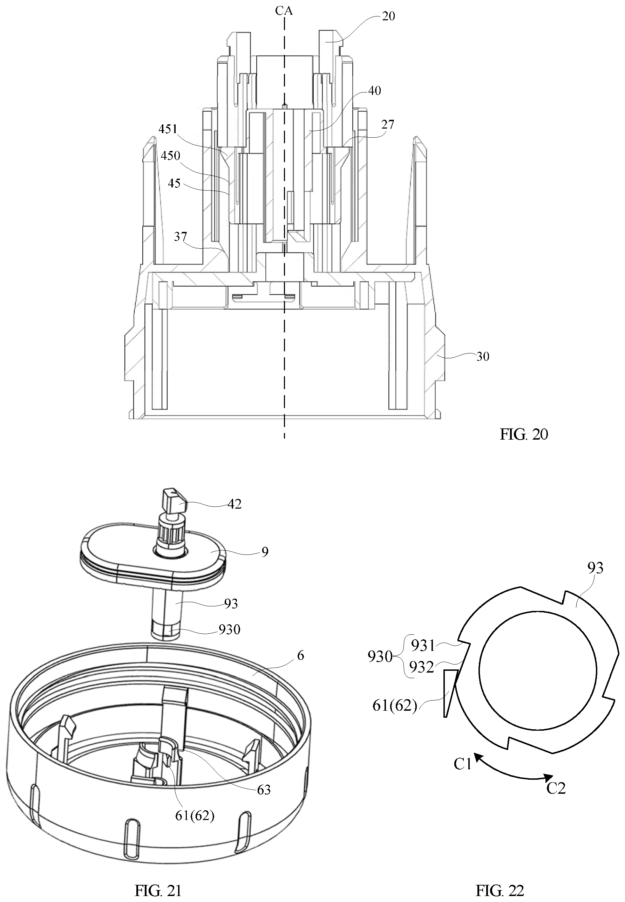

FIG. 18 is an exploded schematic view of the movable body 20 and the penetrating member 40 from a first perspective according to an example of the present disclosure. FIG. 19 is an exploded schematic view of the movable body 20 and the penetrating member 40 from a second perspective according to an example of the present disclosure. FIG. 20 is a cross-sectional schematic view illustrating the penetrating member 40 in a retained state according to an example of the present disclosure.

In some examples, the movable body 20 and the penetrating member 40 may be integrally and fixedly connected to one another. In other examples, the movable body 20 and the penetrating member 40 may be detachably fitted and combined. In some examples, the penetrating member 40 may be releasably retained by the movable body 20.

In some examples, the movable body 20 may comprise a hollow portion 26 (see FIG. 18). More specifically, the sidewall 22 of the movable body 20 and the first bottom portion 25 facing the proximal end 101 may cooperate to define the hollow portion 26. In some examples, the penetrating member 40 may be releasably disposed within the hollow portion 26 of the movable body 20. In some examples, when released, the penetrating member 40 may move within the hollow portion 26 of the movable body 20. In such a case, the hollow portion 26 is formed by the first bottom portion 25 and the sidewall, the receiving portion 21 is provided on the first bottom portion 25, and the penetrating member 40 is disposed within the hollow portion 26, thereby allowing the penetrating member 40 to move relative to the receiving portion 21 once released. Accordingly, after the monitoring device 800 has been applied to the host, the penetrating member 40 may be released and move relative to the receiving portion 21, thereby allowing the penetrating member 40 to withdraw from the host.

In some examples, the movable body 20 may comprise a second through-hole 260 connecting the receiving portion 21 and the hollow portion 26 (see FIG. 19). In some examples, the penetrating member 40 may contact the monitoring device 800 through the second through-hole 260. Accordingly, during application, the penetrating member 40 may conveniently introduce at least a portion of the monitoring device 800 beneath the skin of the host.

In other examples, the penetrating member 40 may be fixedly provided to the movable body 20. In such a case, after the monitoring device 800 has been applied to the host, the movable body 20 may be manually withdrawn as a whole from the host, thereby causing the penetrating member 40 to also withdraw from the host.

In some examples, the second drive portion 52 may be disposed between the movable body 20 and the penetrating member 40. In some examples, the second drive portion 52 may be disposed between the first bottom portion 25 and the penetrating member 40. The second drive portion 52 may be configured to apply an action to the penetrating member 40 toward the distal end 102. When the penetrating member 40 is released, the second drive portion 52 may apply an action to the penetrating member 40 toward the distal end 102 so as to withdraw the penetrating member 40 from the host. Accordingly, the timing at which the penetrating member 40 withdraws from the host may be controlled more accurately.

In some examples, the second drive portion 52 may have an energy-storing state and an energy-releasing state. When the second drive portion 52 switches from the energy-storing state to the energy-releasing state, the second drive portion 52 releases energy and applies an action to the penetrating member 40 toward the distal end 102. When the penetrating member 40 is retained, the second drive portion 52 may reside between the movable body 20 and the penetrating member 40 in the energy-storing state. When the penetrating member 40 is released, the second drive portion 52 may switch from the energy-storing state to the energy-releasing state and apply an action to the penetrating member 40 toward the distal end 102.

In some examples, the second drive portion 52 may have a compressed state and an expanded state. When the second drive portion 52 switches from the compressed state to the expanded state, the second drive portion 52 may release energy. In some examples, the second drive portion 52 may be an elastic element. In some examples, opposite ends of the second drive portion 52 may respectively abut against the first bottom portion 25 and the penetrating member 40. In other words, opposite ends of the second drive portion 52 may respectively abut against the movable body 20 and the penetrating member 40. When the penetrating member 40 is retained, the second drive portion 52 may be in the compressed state. In such a case, when the penetrating member 40 is released, the compressed second drive portion 52 may automatically apply an action force to the penetrating member 40, thereby automatically retracting the penetrating member 40 and facilitating use by an operator. In some examples, the second drive portion 52 may be a spring.

In some examples, as described above, the penetrating member 40 may comprise the needle portion 41 and the carrying portion 42 connected to the needle portion 41 (see FIG. 18). In some examples, the needle portion 41 may comprise a groove extending along a length direction of the needle portion 41. The sensor 8 may be disposed at least partially within the groove of the needle portion 41. In such a case, the sensor 8 may be received within the groove so that the sensor 8 follows the needle portion 41 when being introduced beneath the skin of the host.

In some examples, the penetrating member 40 may comprise a support seat 43 configured to support the needle portion 41 (see FIG. 18). The support seat 43 may support the needle portion 41 via the carrying portion 42.

In some examples, the support seat 43 may be disposed within the hollow portion 26 of the movable body 20, and the carrying portion 42 and/or the needle portion 41 may extend through the second through-hole 260. In such a case, by extending the carrying portion 42 and/or the needle portion 41 through the second through-hole 260, the carrying portion 42 and/or the needle portion 41 may engage the monitoring device 800 accommodated within the receiving portion 21, thereby facilitating introduction of at least a portion of the monitoring device 800 beneath the skin of the host through the penetrating member 40.

In some examples, the movable body 20 may comprise a third positioning portion 252 (see FIG. 18). The third positioning portion 252 may be configured to position the second drive portion 52. In such a case, the third positioning portion 252 may more stably define the location at which the second drive portion 52 applies an action to the penetrating member 40. In some examples, the third positioning portion 252 may be provided on the first bottom portion 25.

In some examples, one end of the second drive portion 52 may be disposed around the third positioning portion 252 of the first bottom portion 25. In some examples, the third positioning portion 252 may have a cylindrical shape. In such a case, one end of the first drive portion 51 may be disposed around the third positioning portion 252. In some examples, the penetrating member 40 may comprise a fourth positioning portion 44 (see FIG. 19). In some examples, the fourth positioning portion 44 may comprise a hollow cylindrical protrusion formed on the support seat 43. In such a case, the end of the second drive portion 52 that contacts the penetrating member 40 may be disposed around or nested within the fourth positioning portion 44. In some examples, the third positioning portion 252 and/or the fourth positioning portion 44 may each be formed by at least two arcuate column segments. In such a case, an end of the first drive portion 51 may be inserted between adjacent arcuate column segments.

In some examples, the needle portion 41 and the support seat 43 may be integrally formed. In other examples, the needle portion 41 and the support seat 43 may be detachably fitted and combined. Accordingly, the needle portion 41 may be sterilized separately with greater convenience.

In some examples, male and female mechanical fit structures may be provided on an inner wall of the movable body 20 and an outer wall of the penetrating member 40. In some examples, the movable body 20 may comprise a fifth engagement feature 29, and the penetrating member 40 may comprise a sixth engagement feature 46 (see FIGS. 18 and 19). The fifth engagement feature 29 and the sixth engagement feature 46 may cooperate to form the male and female mechanical fit structures. In some examples, the male and female mechanical fit structures may comprise protrusion-and-groove cooperation structures. In some examples, the fifth engagement feature 29 may comprise one or more rib-like protrusions provided on the inner wall of the movable body 20, and the sixth engagement feature 46 may comprise one or more rib-like grooves provided on the outer wall of the penetrating member 40. When the penetrating member 40 is fitted into the movable body 20, the one or more rib-like protrusions of the movable body 20 may respectively fit into the one or more rib-like grooves of the penetrating member 40. In this case, cooperation between the rib-like protrusions and the rib-like grooves facilitates fitting of the movable body 20 and the penetrating member 40, guides the direction of relative movement between the penetrating member 40 and the movable body 20, and helps suppress undesired rotation of the penetrating member 40. In some examples, a plurality of rib-like protrusions provided on the inner wall of the movable body 20 may be uniformly or symmetrically distributed around the inner wall of the movable body 20.

In some examples, as described above, the penetrating member 40 may be releasably retained by the movable body 20. In some examples, the movable body 20 may comprise a second retaining portion 27 (see FIG. 18). The second retaining portion 27 may be configured to releasably retain the penetrating member 40. In some examples, the second retaining portion 27 may be formed on the sidewall 22 of the movable body 20. In some examples, the second retaining portion 27 may comprise a notch formed in the sidewall 22 of the movable body 20 (see FIG. 18).

In some examples, the penetrating member 40 may comprise a second locking portion 45. The second locking portion 45 may be configured to releasably interlock with the second retaining portion 27. Accordingly, the penetrating member 40 may be releasably retained by the second retaining portion 27 through cooperation between the second locking portion 45 and the second retaining portion 27. In some examples, the second locking portion 45 may be provided on the support seat 43 of the penetrating member 40.

In some examples, the second locking portion 45 may comprise an arm 450 extending substantially along the direction of the central axis CA (hereinafter referred to as a fourth arm 450 for ease of reference) and a protrusion 451 linked with the fourth arm 450 and projecting away from the central axis CA in a direction substantially orthogonal to the central axis CA (hereinafter referred to as a fourth protrusion 451 for ease of reference). In such a case, when the penetrating member 40 is disposed within the hollow portion 26 of the movable body 20, the second locking portion 45 may engage the notch of the movable body 20, thereby retaining the penetrating member 40 while maintaining a simplified structure.

In some examples, the fourth arm 450 may be elastic in a direction substantially orthogonal to the central axis CA. In some examples, when the penetrating member 40 is retained, the fourth protrusion 451 may extend through the notch. In such a case, retention of the second locking portion 45 may be achieved in a simplified structure by engaging the fourth protrusion 451 with the notch formed in the movable body 20.

In some examples, the second retaining portion 27 may comprise a first notch 271 and a second notch 272 that are interconnected and extend substantially along the central axis CA (see FIG. 18). The first notch 271 may be located closer to the proximal end 101, and the second notch 272 may be located closer to the distal end 102. In some examples, the widths of the first notch 271 and the second notch 272 may differ. For example, the width of the first notch 271 may be greater than the width of the second notch 272.

In some examples, the first notch 271 may comprise a surface facing the proximal end 101. In some examples, the fourth protrusion 451 may comprise a surface facing the distal end 102. When the penetrating member 40 is retained by the second retaining portion 27, the surface facing the distal end 102 of the fourth protrusion 451 engages the surface facing the proximal end 101 of the first notch 271.

In some examples, when an action is applied to the fourth arm 450 in a direction substantially orthogonal to the central axis CA, the fourth arm 450 may pivot. For example, when an action directed toward the central axis CA is applied to the fourth arm 450 in a direction substantially orthogonal to the central axis CA, the fourth arm 450 may deflect such that an end of the fourth arm 450 proximal to the distal end 102 moves toward the central axis CA. When the fourth arm 450 deflects toward the central axis CA to a position at which the second locking portion 45 is separated from the second retaining portion 27, the penetrating member 40 may be released. Accordingly, the penetrating member 40 may be released in a simple and convenient manner.

In some examples, when the penetrating member 40 is released, the arm of the second locking portion 45 may be compressed toward the central axis CA. Accordingly, the second locking portion 45 may be conveniently released. In some examples, the second retaining portion 27 and the second locking portion 45 may be releasably interlocked through at least one structure of a latch, hook, catch, or pin. Thus, an interlocking manner that is easy to release may be provided.

In some examples, the second locking portion 45 of the penetrating member 40 may be formed as a shoulder structure (see FIGS. 18, 19, and 20). In some examples, the fourth arm 450 may gradually extend away from the central axis CA in the direction of the distal end 102. In some examples, when projected along the direction of the central axis CA, a projected area of the fourth protrusion 451 may completely or partially overlap a projected area of the first notch 271. In some examples, one or more second locking portions 45 may be provided, for example one, two, three, or four. In some examples, the number of second locking portions 45 may correspond to the number of second retaining portions 27. In some examples, the width of the distal-facing surface of the fourth protrusion 451 may be smaller than the width of the first notch 271 and greater than the width of the second notch 272.

In some examples, the second housing 30 may comprise a second trigger portion 37 (see FIGS. 11, 16, and 20). The second trigger portion 37 may be configured to separate the second retaining portion 27 from the second locking portion 45 so as to release the penetrating member 40. Accordingly, the second trigger portion 37 facilitates release of the movable body 20. In some examples, the second trigger portion 37 may apply an action directed toward the central axis CA to the second locking portion 45 so as to compress the second locking portion 45 inwardly, thereby separating the second locking portion 45 from the second retaining portion 27. In some examples, after the movable body 20 and the penetrating member 40 have moved toward the proximal end 101 to a predetermined position, the second locking portion 45 may disengage from the second retaining portion 27, thereby releasing the penetrating member 40. Accordingly, release of the penetrating member 40 may be automatically triggered when the movable body 20 and the penetrating member 40 reach the predetermined position, thereby enabling more precise control of the timing at which the penetrating member 40 withdraws from the host. Here, the predetermined position may be a position at which the monitoring device 800 has already been applied to the body surface of the host. In some examples, the predetermined position may alternatively be a position at which the penetrating member 40 has introduced the implanted portion 81 of the sensor 8 to a predetermined depth beneath the skin of the host. In other words, the penetrating member 40 may be configured to be released after moving a predetermined distance relative to the second housing 30.

In some examples, the second trigger portion 37 may be formed on an inner wall of the second housing 30. In some examples, the second trigger portion 37 may comprise a protrusion formed on the inner wall of the second housing 30 and projecting toward the central axis CA in a direction substantially perpendicular thereto (hereinafter referred to as a fifth protrusion for ease of reference). When the movable body 20 and the penetrating member 40 move relative to the second housing 30 (i.e., when the movable body 20 and the penetrating member 40 move toward the proximal end 101), the second locking portion 45 first reaches a position at which it contacts the second trigger portion 37. As the movable body 20 and the penetrating member 40 continue moving toward the proximal end 101, the fifth protrusion may abut the fourth protrusion 451 and continuously apply an action thereto toward the central axis CA. When the movable body 20 and the penetrating member 40 reach the predetermined position, the fourth arm 450 contracts inwardly until the fourth protrusion 451 separates from the second retaining portion 27, thereby releasing the penetrating member 40.

The cooperation among the second retaining portion 27, the second locking portion 45, and the second trigger portion 37 will now be described again. When the penetrating member 40 is retained by the second retaining portion 27, the second locking portion 45 contacts the second retaining portion 27, and the second retaining portion 27 restricts movement of the second locking portion 45 toward the distal end 102, thereby retaining the penetrating member 40 (see FIG. 20). As the movable body 20 and the penetrating member 40 move toward the proximal end 101, the second locking portion 45 first reaches a position at which it contacts the second trigger portion 37. As movement continues, the fifth protrusion abuts the fourth protrusion 451 and continuously applies an action thereto toward the central axis CA. When the movable body 20 and the penetrating member 40 reach the predetermined position, the fourth arm 450 contracts inwardly until the fourth protrusion 451 separates from the second retaining portion 27, thereby releasing the penetrating member 40.

In some examples, after the penetrating member 40 is released by the movable body 20, the second drive portion 52 drives the penetrating member 40 toward the distal end 102 so that the penetrating member 40 withdraws from the host. In some examples, the retracted penetrating member 40 may be entirely located within the internal space of the first housing 10. In such a case, this arrangement prevents the sharp needle portion 41 from being exposed outside the applicator device 100, thereby enhancing safety of the applicator device 100.

In some examples, as described above, the movable body 20 may comprise the receiving portion 21. In some examples, the receiving portion 21 may be provided at an end of the movable body 20 facing the proximal end 101, and an opening of the receiving portion 21 may face the proximal end 101 (see FIG. 19). In such a case, this arrangement facilitates installation of the monitoring device 800 into the receiving portion 21.

In some examples, the receiving portion 21 may comprise one or more constraining portions 210 configured to constrain the monitoring device 800 (see FIG. 19). In this case, the monitoring device 800 accommodated within the receiving portion 21 may be constrained more securely by the constraining portions 210.

In some examples, each constraining portion 210 may comprise a constraining arm 211 and a protruding portion 212 extending toward the central axis CA in a direction substantially perpendicular thereto (see FIG. 19). Here, the constraining arm 211 may extend along the direction of the central axis CA. In some examples, the constraining arm 211 may be elastic. In some examples, the constraining arm 211 may gather inwardly toward the central axis CA. In such a case, the constraining portion 210 may securely constrain a monitoring device 800 having an outer contour smaller than the receiving portion 21. For example, the electronic assembly 9 of the monitoring device 800 may be inserted within the receiving portion 21 such that an interference fit is formed between the constraining portion 210 and the enclosure 91 of the electronic assembly 9. In some examples, one or more constraining portions 210 may be provided, for example one, two, three, or four. In some examples, the constraining portions 210 may be uniformly distributed around the periphery of the receiving portion 21.

FIG. 21 is a schematic view illustrating the sealing assembly 93 and the applicator cap 6 according to an example of the present disclosure.

In some examples, the applicator device 100 may comprise an applicator cap 6 (see FIG. 9). In some examples, the applicator cap 6 may be detachably fitted to the first housing 10. When the applicator cap 6 is fitted to the first housing 10, the second housing 30 may be located within a sealed accommodation space defined by the applicator cap 6 and the first housing 10. In such a case, cooperation between the applicator cap 6 and the first housing 10 forms a sealed space for protecting internal structures, and placement of the second housing 30 within the sealed space helps prevent unintended actuation, thereby improving the reliability of the applicator device 100.

In some examples, the applicator cap 6 may be removed from the first housing 10 so that the applicator device 100 is placed in a ready-to-apply state. As described above, the monitoring device 800 may comprise a sealing assembly 93 configured to seal the sensor 8. In some examples, the applicator cap 6 may be removed from the first housing 10, and the sealing assembly 93 may be removed from the monitoring device 800, thereby placing the applicator device 100 in the ready-to-apply state.

In some examples, the applicator cap 6 and the first housing 10 may be connected by threads. More specifically, an outer peripheral surface of an end of the first housing 10 adjacent the host may be provided with external threads, and an inner wall of the applicator cap 6 may be provided with internal threads corresponding to the external threads. Such an arrangement facilitates attachment and removal of the applicator cap 6 relative to the first housing 10. In some examples, the applicator cap 6 may be rotated in a predetermined direction to remove it from the first housing 10 and rotated in a direction opposite to the predetermined direction to mount it onto the first housing 10. In some examples, the predetermined direction may be a clockwise direction or a counterclockwise direction.

In some examples, the sealing assembly 93 may comprise a first engagement feature 930, and the applicator cap 6 may comprise a second engagement feature 61 (see FIG. 21). Cooperation between the first engagement feature 930 and the second engagement feature 61 causes the sealing assembly 93 to rotate together with the applicator cap 6 when the applicator cap 6 is rotated in a first direction, while allowing the sealing assembly 93 to remain stationary when the applicator cap 6 is rotated in a second direction opposite to the first direction. In such a case, the sealing assembly 93 can be removed simultaneously with removal of the applicator cap 6 by rotating the applicator cap 6 in the first direction, thereby facilitating use. Furthermore, during mounting of the applicator cap 6 onto the first housing 10 by rotating the applicator cap 6 in the second direction, the sealing condition of the sealing assembly 93 is not affected, thereby facilitating factory assembly of the applicator device 100 and the monitoring device 800.

FIG. 22 is a schematic view illustrating a structure of an inclined slot according to an example of the present disclosure. FIG. 22 is a projection view taken along the direction of the central axis CA of the applicator device 100. For clarity in illustrating cooperation between the sealing assembly 93 and the applicator cap 6, certain components and lines that could obscure or complicate the illustration have been omitted.

In some examples, the first engagement feature 930 may comprise an inclined slot formed in a sidewall of the sealing assembly 93 (see FIG. 22). The inclined slot may comprise a stop surface 931 and an inclined surface 932 transitioning from the sidewall of the sealing assembly 93. The second engagement feature 61 may comprise a protrusion 62 corresponding to the inclined slot (also referred to as a sixth protrusion 62 for ease of reference) (see FIGS. 21 and 22). When the applicator cap 6 is rotated in the first direction, the sixth protrusion 62 may abut the stop surface 931 so that the sealing assembly 93 rotates together with the applicator cap 6. When the applicator cap 6 is rotated in the second direction opposite to the first direction, the sixth protrusion 62 may slide along the inclined surface 932 and the outer peripheral surface of the sidewall of the sealing assembly 93. Accordingly, cooperation between the first engagement feature 930 and the second engagement feature 61 achieves the intended function described above.

Referring to FIG. 22, in the illustrated example of FIG. 22, direction C1 schematically represents a clockwise direction and direction C2 schematically represents a counterclockwise direction (i.e., opposite to direction C1). When the applicator cap 6 is rotated in direction C1, the sixth protrusion 62 of the applicator cap 6 may abut the stop surface 931. When the applicator cap 6 is rotated in direction C2, the sixth protrusion 62 may slide along the inclined surface 932 and the outer peripheral surface of the sidewall of the sealing assembly 93.

In some examples, the applicator cap 6 may comprise a fourth restricting portion 63 (see FIG. 21). The fourth restricting portion 63 may restrict movement of the sealing assembly 93. In some examples, the fourth restricting portion 63 may surround an outer periphery of the sealing assembly 93 so as to restrain the sealing assembly 93. Such a configuration helps suppress undesired movement or wobbling of the sealing assembly 93 and the monitoring device 800 within the first housing 10. In some examples, the sealing assembly 93 may also be referred to as a sensor cap.

In some examples, the fourth restricting portion 63 may comprise an arm extending along the direction of the central axis CA (which may be referred to as a fifth arm for ease of reference). In some examples, the second engagement feature 61 may be formed on the fourth restricting portion 63. Accordingly, the second engagement feature 61 can conveniently engage the first engagement feature 930 while the fourth restricting portion 63 limits the sealing assembly 93 in position.

In some examples, after the applicator cap 6 has been removed, the monitoring device 800 may be conveniently applied to a host using the applicator device 100. In some examples, a desiccant may be disposed within the sealed accommodation space defined by the applicator cap 6 and the first housing 10. Such an arrangement helps create a dry environment within the applicator device 100, thereby promoting preservation of the monitoring device 800.

The application process performed by the applicator device 100 will now be described again. Application of the monitoring device 800 to a host using the applicator device 100 may comprise an application stage and a retraction stage. During the application stage, the applicator device 100 is applied to the host. During the retraction stage, the penetrating member 40 is withdrawn from the host.

First, the applicator cap 6 is removed from the first housing 10 so that the applicator device 100 enters a ready-to-apply state.

During the application stage, the applicator device 100 is placed against a body surface of the host such that the proximal surface 31 of the second housing 30 contacts the body surface of the host. The first housing 10 is then pressed toward the host. When the first trigger portion 33 reaches the first locking portion 23, the first trigger portion 33 actuates the first locking portion 23 to pivot toward the central axis CA, thereby separating the first locking portion 23 from the first retaining portion 12 and releasing the movable body 20. Subsequently, the first drive portion 51 (or manual force) applies a force to the movable body 20 toward the proximal end 101, causing the movable body 20 and the penetrating member 40 to move together toward the proximal end 101. The monitoring device 800 accommodated within the receiving portion 21 is thereby advanced against the body surface of the host, and under the action of the penetrating member 40, at least a portion of the sensor 8 is positioned beneath the skin of the host.

During the retraction stage, as the movable body 20 and the penetrating member 40 move toward the proximal end 101, the second trigger portion 37 disposed on the inner wall of the second housing 30 contacts the second locking portion 45 of the penetrating member 40. The second trigger portion 37 actuates the second locking portion 45 to deflect toward the central axis CA, thereby separating the second locking portion 45 from the second retaining portion 27 and releasing the penetrating member 40. Subsequently, the second drive portion 52 (or manual force) applies an action to the penetrating member 40 toward the distal end 102, causing the penetrating member 40 to move toward the distal end 102 and withdraw from the host.

It should additionally be noted that, during the retraction stage, the first drive portion 51 (or manual force) may continue to apply an action to the movable body 20 toward the proximal end 101. In other words, where the first drive portion 51 comprises an elastic element (for example, a spring), the first drive portion 51 may remain in an energy-storing state even after the movable body 20 has been driven toward the host and the monitoring device 800 accommodated within the receiving portion 21 has been adhered to the body surface of the host. In such a case, during the retraction stage, the monitoring device 800 remains pressed against the body surface of the host under the action of the first drive portion 51, thereby effectively reducing the possibility that the monitoring device 800 may detach from the host as a result of separation of the penetrating member 40 from the sensor 8 during retraction.

Accordingly, the present disclosure provides an applicator device 100 and an analyte monitoring system 1000 that are easy to operate and capable of accurately applying the monitoring device 800 to a host.

Although the present disclosure has been described in detail above with reference to the accompanying drawings and specific embodiments, it will be understood that the foregoing description does not limit the present disclosure in any way. Those skilled in the art may make various modifications and variations as needed without departing from the spirit and scope of the present disclosure, and all such modifications and variations are intended to fall within the scope of the present disclosure.

## Claims

1. An applicator device for applying a monitoring device to a host, **characterized in that**:
the applicator device comprises a first housing, a second housing, a drive mechanism, a movable body, a penetrating member disposed on the movable body, a proximal end configured to be located proximal to the host during operation, and a distal end configured to be located distal to the host during operation,
wherein the first housing comprises a first retaining portion configured to releasably retain the movable body, and the second housing comprises a proximal surface configured to contact skin of the host during operation;
wherein the movable body comprises a receiving portion configured to accommodate the monitoring device, and the penetrating member is configured to introduce at least a portion of the monitoring device beneath the skin of the host;
and wherein the first housing and the second housing are configured to be moved relative to one another toward one another so as to release retention of the movable body by the first retaining portion, and after the movable body is released, the movable body and the penetrating member are moved together toward the proximal end under an action of the drive mechanism to apply the monitoring device to the host, with at least a portion of the monitoring device positioned beneath the skin of the host.

2. The applicator device according to claim 1, **characterized in that**:
wherein the movable body comprises a first locking portion configured to releasably interlock with the first retaining portion, and
wherein the second housing comprises a first trigger portion configured to separate the first retaining portion from the first locking portion so as to release the movable body.

3. The applicator device according to claim 2, **characterized in that**:
wherein, when the first housing and the second housing move relative to each other along a direction of a central axis of the applicator device, the first trigger portion acts on the first locking portion so as to separate the first locking portion from the first retaining portion.

4. The applicator device according to claim 3, **characterized in that**:
wherein the first retaining portion is a protruding structure formed on an inner wall of the first housing,
wherein, when the movable body is retained by the first retaining portion, the first locking portion engages the protruding structure, and
wherein the first trigger portion causes the first locking portion to deflect in a direction away from the first retaining portion so as to separate the first locking portion from the first retaining portion.

5. The applicator device according to claim 1 or 2, **characterized in that**:
wherein the first housing comprises a first restricting portion configured to inhibit the second housing from disengaging from the first housing,
wherein the second housing comprises a first restricted portion, and
wherein the first restricted portion is coupled to the first restricting portion.

6. The applicator device according to claim 5, **characterized in that**:
wherein the first housing comprises a second restricting portion configured to inhibit movement of the second housing toward the proximal end, and
wherein, after the movable body is released through relative movement between the first housing and the second housing, the second housing is restricted by the second restricting portion.

7. The applicator device according to claim 6, **characterized in that**:
wherein the first restricting portion and the second restricting portion are arranged adjacent to one another along a direction of a central axis of the applicator device, and
wherein the first restricted portion is configured to cooperate with the second restricting portion to retain the second housing.

8. The applicator device according to claim 1 or 2, **characterized in that**:
wherein the drive mechanism comprises a first drive portion and a second drive portion,
wherein the movable body comprises a second retaining portion configured to releasably retain the penetrating member,
wherein, after the movable body and the penetrating member are moved together toward the proximal end to a predetermined position under an action of the first drive portion, the second retaining portion releases the penetrating member,
and wherein the penetrating member moves toward the distal end under an action of the second drive portion.

9. The applicator device according to claim 8, **characterized in that**:
wherein the first drive portion is an elastic element,
wherein opposite ends of the first drive portion respectively abut against the first housing and the movable body,
and wherein, when the movable body is retained by the first retaining portion, the first drive portion is in a compressed state.

10. The applicator device according to claim 8, **characterized in that**:
wherein the second drive portion is an elastic element,
wherein opposite ends of the second drive portion respectively abut against the movable body and the penetrating member,
and wherein, when the penetrating member is retained by the second retaining portion, the second drive portion is in a compressed state.

11. The applicator device according to claim 1, **characterized in that**:
wherein the applicator device further comprises an applicator cap detachably mounted onto the first housing,
and wherein, when the applicator cap is mounted onto the first housing, the second housing is located within a sealed accommodation space defined by the applicator cap and the first housing.

12. The applicator device according to claim 11, **characterized in that**:
wherein the monitoring device comprises:
a sensor configured to be partially placed beneath the skin of the host;
an electronic assembly coupled to the sensor; and
a sealing assembly configured to seal the sensor,
wherein the applicator cap is coupled to the sealing assembly,
and wherein, when the applicator cap is removed from the first housing, the sealing assembly is removed together with the applicator cap to place the applicator device in a ready-to-apply state.

13. The applicator device according to claim 12, **characterized in that**:
wherein the sealing assembly comprises a first engagement feature,
wherein the applicator cap comprises a second engagement feature,
and wherein the first engagement feature and the second engagement feature cooperate such that, when the applicator cap is rotated in a first direction, the sealing assembly rotates together with the applicator cap, and when the applicator cap is rotated in a second direction opposite to the first direction, the sealing assembly remains stationary.

14. An analyte monitoring system, **characterized in that**:
the analyte monitoring system comprises an applicator device according to any one of claims 1-13; and
a monitoring device.
